# EUROPEAN PATENT SPECIFICATION

(11) **EP 1 127 126 B1**
(45) Date of publication and mention of the grant of the patent: **18.10.2006**
(21) Application number: 99960200.6
(22) Date of filing: 05.11.1999
(51) Int. Cl.: C12N 15/12, C07K 14/705, C12Q 1/68, C07K 16/28

(54) **G-PROTEIN COUPLED RECEPTORS, HOMOLOGOUS TO EBV-INDUCED GPCR 2 (EBI-2). METHODS TO SEEK FOR LIGANDS THEREOF**
G-PROTEIN-GEKOPPELTER REZEPTOREN, DIE HOLOMOG ZU EBV-INDUZIERTER GPCR (EBI-2) SIND, VERFAHREN ZUR IDENTIFIZIERUNG VON IHREN ENTSPRECHENDEN LIGANDEN
RECEPTEURS A COUPLAGE DE PROTEINE G, HOMOLOGUES DE GPCR 2 INDUITS PAR EBV (EBI-2), ET PROCEDES PERMETTANT DE RECHERCHER CERTAINS DE LEURS LIGANDS

(30) Priority: 06.11.1998 US 187134; 25.08.1999 US 382918
(43) Date of publication of application: 29.08.2001
(73) Proprietor: Bayer HealthCare AG, 51368 Leverkusen (DE)
(72) Inventor: GLUCKSMANN, Maria, Alexandra, Lexington, MA 02173 (US); GU, Wei, Brookline, MA 02445 (US); WEICH, Nadine, S., Brookline, MA 02446 (US)
(74) Representative: Spaltmann, Frank
(86) International application number: PCT/US1999/025956
(87) International publication number: WO 2000/028028

(56) References cited:
- WO-A-98/11218
- WO-A-98/45467
- WO-A-98/50549
- DATABASE EMBL [Online] EBI, Hinxton, UK AC : AA274112, 29 March 1997 (1997-03-29) MARRA, M. ET AL.: "The WashU-HHMI Mouse EST Project" XP002133577
- DATABASE EMBL [Online] EBI, Hinxton, UK AA447306, 7 April 1998 (1998-04-07) NELSON, P.S. ET AL.: "An expressed sequence-tag-database of the humanprostate : sequence analysis of 1168 cDNA clones" XP002133578

### SEQUENCE LISTING

<110> Glucksmann, Maria A.
   Gu, Wei
<120> 15625 Receptor, A Novel G-Protein Coupled Receptor
<130> 5800-13A-1
<140>
   <141>
<160> 5
<170> Patent In Ver. 2.0
<210> 1
   <211> 342
   <212> PRT
   <213> Homo sapiens
<400> 1
<210> 2
   <211> 2286
   <212> DNA
   <213> Homo sapiens
<400> 2
   cgtccgtagc tttgagtcca gtgtttgaag acaatctctg attgtgaagc cctctttttc 60
   tctccttcta tttctctcta gagcactcaa gactttactg acgaaaactc aggaaatcct 120
   ctatcacaaa gaggtttggc aactaaacta agacattaaa aggaaaatac cagatgccac 180
   tctgcaggct gcaataacta ctacttactg gatacattca aaccctccag aatcaacagt 240
   tatcaggtaa ccaacaagaa atgcaagccg tcgacaacct cacctctgcg cctgggacca 300
   ccagtctgtg caccagagac tacaaaatca cccaggtcct cttcccactg ctctacactg 360
   tcctgttttt tgttggactt atcacaaatg gcctggcgat gaggattttc tttcaaatcc 420
   ggagtaaatc aaactttatt atttttctta agaacacagt catttctgat cttctcatga 480
   ttctgacttt tccattcaaa attcttagtg atgccaaact gggaacagga ccactgagaa 540
   cttttgtgtg tcaagttacc tccgtcatat tttatttcac aatgtatatc agtatttcat 600
   tcctgggact gataactatc gatcgctacc agaagaccac caggccattt aaaacatcca 660
   accccaaaaa tctcttgggg gctaagattc tctctgttgt catctgggca ttcatgttct 720
   tactctcttt gcctaacatg attctgacca acaggcagcc gagagacaag aatgtgaaga 780
   aatgctcttt ccttaaatca gagttcggtc tagtctggca tgaaatagta aattacatct 840
   gtcaagtcat tttctggatt aatttcttaa ttgttattgt atgttataca ctcattacaa 900
   aagaactgta ccggtcatac gtaagaacga ggggtgtagg taaagtcccc aggaaaaagg 960
   tgaacgtcaa agttttcatt atcattgctg tattctttat ttgttttgtt cctttccatt 1020
   ttgcccgaat tccttacacc ctgagccaaa cccgggatgt ctttgactgc actgctgaaa 1080
   atactctgtt ctatgtgaaa gagagcactc tgtggttaac ttccttaaat gcatgcctgg 1140
   atccgttcat ctattttttc ctttgcaagt ccttcagaaa ttccttgata agtatgctga 1200
   agtgccccaa ttctgcaaca tctctgtccc aggacaatag gaaaaaagaa caggatggtg 1260
   gtgacccaaa tgaagagact ccaatgtaaa caaattaact aaggaaatat ttcaatctct 1320
   ttgtgttcag aactcgttaa agcaaagcgc taagtaaaaa tattaactga cgaagaagca 1380
   actaagttaa taataatgac tctaaagaaa cagaagatta caaaagcaat tttcatttac 1440
   ctttccagta tgaaaagcta tcttaaaata tagaaaacta atctaaactg tagctgtatt 1500
   agcagcaaaa caaacgacat ccaattgtca tgctgcatgc aaaactacac agaattcatg 1560
   ttttgcagag ttttgccaaa atgagtaatc atataatatt tactgtaatt tttaaaatac 1620
   attatcgttc acaattttat tttttcataa tcaactaagg aagaacgatc aattggatat 1680
   aatttcttac caaaaatgat agttaaaatg tatatatatc ctagtcccct aaccaaatcc 1740
   tgacctattg ggatacttat aaaaatttaa gtaagtggga tacacaaaga ataataacta 1800
   ttaacttttc attattagca aaaacctaag ggatttaaac taattgaaac tgtatttgat 1860
   tggacttaat tttttatgtt tatttagaag ataaagattt aaagaagacc tttacaataa 1920
   agagaagaaa tatcgaagtc attaaaataa ggagacttac ttttatgaca ttctaatact 1980
   aaaaaatata gaaatatttc cttaattcta gagaaactag ttttactaat tttttacaac 2040
   ttcaataata ccatcactga cacttacctt tattaattag cttctagaaa atagctgcta 2100
   attaggttaa tgaacatttt accttagtga aaaaaattaa ttaaatatga ttacaaagtt 2160
   gcacagcata actactgaga ggaaagtgat tgatctgttt gtaattactt gtttgtattg 2220
   gtgtgtataa aatacaaaat ttacattaaa ctctaaaaaa aaaaaaaaaa aaaaaaaaaa 2280
   gggcgg                                                            2286
<210> 3
   <211> 342
   <212> PRT
   <213> Macaca sp.
<400> 3
   Met Gln Ala Ile Asp Asn Leu Thr Ser Ala Pro Gly Asn Thr Ser Leu
   1               5                   10                  15
   Cys Thr Arg Asp Tyr Lys Ile Thr Gln Val Leu Phe Pro Leu Leu Tyr
               20                  25                  30
   Thr Val Leu Phe Phe Val Gly Leu Ile Thr Asn Ser Leu Ala Met Arg
           35                  40                  45
   Ile Phe Phe Gln Ile Arg Ser Lys Ser Asn Phe Ile Ile Phe Leu Lys
       50                  55                  60
   Asn Thr Val Ile Ser Asp Leu Leu Met Ile Leu Thr Phe Pro Phe Lys
   65                  70                  75                  80
   Ile Leu Ser Asp Ala Lys Leu Gly Thr Gly Pro Leu Arg Thr Phe Val
                   85                  90                  95
   Cys Gln Val Thr Ser Val Ile Phe Tyr Phe Thr Met Tyr Ile Ser Ile
               100                 105                 110
   Ser Phe Leu Gly Leu Ile Thr Ile Asp Arg Tyr Gln Lys Thr Thr Arg
           115                 120                 125
   Pro Phe Lys Thr Ser Asn Pro Lys Asn Leu Leu Gly Ala Lys Ile Leu
       130                 135                 140
   Ser Val Leu Ile Trp Ala Phe Met Phe Leu Leu Ser Leu Pro Asn Met
   145                 150                 155                 160
   Ile Leu Thr Asn Arg Arg Pro Arg Asp Lys Asn Val Lys Lys Cys Ser
                   165                 170                 175
   Phe Leu Lys Ser Glu Phe Gly Leu Val Trp His Glu Ile Val Asn Tyr
               180                 185                 190
   Ile Cys Gln Val Ile Phe Trp Ile Asn Phe Leu Ile Val Ile Val Cys
           195                 200                 205
   Tyr Thr Leu Ile Thr Lys Glu Leu Tyr Arg Ser Tyr Val Arg Thr Arg
       210                 215                 220
   Gly Val Gly Lys Val Pro Arg Lys Lys Val Asn Val Lys Val Phe Ile
   225                 230                 235                 240
   Ile Ile Ala Val Phe Phe Ile Cys Phe Val Pro Phe His Phe Ala Arg
                   245                 250                 255
   Ile Pro Tyr Thr Leu Ser Gln Thr Arg Asp Val Phe Asp Cys Ala Ala
               260                 265                 270
   Glu Asn Thr Leu Phe Tyr Val Lys Glu Ser Thr Leu Trp Leu Thr Ser
   275 280 285
   Leu Asn Ala Cys Leu Asp Pro Phe Thr Tyr Phe Phe Leu Cys Lys Ser
   290 295 300
   Phe Arg Asn Ser Leu Ile Ser Met Leu Lys Cys Pro Asn Ser Ala Thr
   305 310 315 320
   Ser Gln Ser Gln Asp Asn Arg Lys Lys Glu Gln Asp Gly Gly Asp Pro
   325 330 335
   Asn Glu Glu Thr Pro Met
   340
<210> 4
   <211> 2272
   <212> DNA
   <213> Macaca sp.
<400> 4
   acgcgtccgc aatctctgat tgtaaagccc tctcttcctc tccttctatt tctctataga 60
   acactcaaga ctttactgat gaaaactcag gaaattctct atcacaaaga ggtttggcaa 120
   ctaaactaag acattaaaag gaaaatacca gatgccactc tgcacgttgc aataactact 180
   atttactgga tacattcaaa tcctccagaa tcaacggtta tcaggtaacc aacaagaaat 240
   gcaagccatc gacaacctca cgtctgcgcc tgggaacacc agtctgtgca ccagagacta 300
   caaaatcacc caggtcctct tcccactgct ctacactgtc ctgttttttg ttggactcat 360
   cacaaatagc ctggcgatga ggattttctt tcaaattcgg agtaaatcaa actttattat 420
   ttttcttaag aacacagtca tttccgatct tctcatgatt ctgacttttc cattcaaaat 480
   tcttagtgat gccaaactgg gaacaggacc actgagaact tttgtgtgtc aagttacctc 540
   cgtcatattt tatttcacaa tgtatatcag tatttcattc ctgggactga taactatcga 600
   tcgctaccag aagaccacca ggccatttaa aacatccaac cccaaaaatc tcttgggggc 660
   taagattctc tctgttctca tctgggcatt catgttctta ctctctttgc ctaacatgat 720
   tctgactaac aggcggccaa gagacaagaa tgtgaagaaa tgctctttcc ttaaatcaga 780
   gttcggccta gtctggcatg aaatagtaaa ttacatctgt caagtcattt tctggattaa 840
   tttcttaatt gtcattgtat gttacacact cattacaaaa gaactgtacc ggtcatatgt 900
   aagaacaagg ggtgtaggta aagtccccag gaaaaaggtg aacgtcaaag ttttcattat 960
   cattgctgta ttctttattt gttttgttcc tttccatttt gcccgaattc cttataccct 1020
   gagccaaacc cgggatgtct ttgactgcgc cgctgaaaat actctgttct atgtgaaaga 1080
   gagtactctg tggttaactt ccttaaatgc atgcctggat ccgttcacct attttttcct 1140
   ttgcaagtcc ttcagaaatt ccttgataag tatgctgaag tgccccaatt ctgcaacatc 1200
   tcagtcccag gacaatagga aaaaagaaca ggatggtggt gacccaaatg aagagactcc 1260
   aatgtaaaca tattaactga ggaaatatgt caatctcttt gcgttcagaa ctcattaaag 1320
   caaagcgcta cgtaaaaata ttaactgacg aagaagcaac tgagttaata acaatgactc 1380
   ttaaaacatg taatagaaga tttacaaaag caattttcat ttacctttcc agtatgaaaa 1440
   gctatgttaa aatatagaaa actaatctaa cctgtagctg tatagtatca aaacaaatga 1500
   catccaattg gcatgctgca tgcaaaacta cacagaattc acgttttgca gagttttgcc 1560
   aaaatgagta atcatataat atctaccgta atgtttaaaa tacattattg ctcacgattt 1620
   tatttcttca taatcaacta aggaagaatt atcaattgga tacaatcttc ttacaaaaaa 1680
   tgacacttaa aatgtatata tatcctagcc cctaaccaaa tcctgaccta ttgggatact 1740
   tataaaaatt tgagtaagtg ggatacacaa agaataataa ctattaactt ttaattatga 1800
   gcaaaaacct aagggttaaa tttaaactaa ttgaaactgt atttgattgg acttaatttt 1860
   tttgtttatt aagaagacac ttgaagaaga cctttacaat aaagagaaga aatatcaaag 1920
   tcattaaaat aaggagagtt acttttatga tattctaaca ctaaacaata tagaaatatt 1980
   tccttaatat tagtttctag agaaactagt tttactaatt ttttacaacc tcaataatac 2040
   catcattgac acttaccttt attaactagc ttctagaaaa tacctgctaa ttaggttaat 2100
   gaacatttta tgttagtgaa aaaaattaat taaatatgat tacaaagttg cacagcataa 2160
   ctactgaaag tgattgatcc atttgtaatt atttgtttgt actggtgtgt ataaaataca 2220
   aaatttacat taaactctaa atcaccaaaa aaaaaaaaaa aaaaaagggc gg         2272
<210> 5
   <211> 269
   <212> PRT
   <213> Unknown
<220>
   <223> Description of Unknown Organism: Rhodopsin family transmembrane receptor
<400> 5
   Gly Asn Ile Leu Val Ile Trp Val Ile Cys Arg Tyr Arg Arg Met Arg
   1               5                   10                  15
   Thr Pro Met Asn Tyr Phe Ile Val Asn Leu Ala Val Ala Asp Leu Leu
               20                  25                  30
   Phe Ser Leu Phe Thr Met Pro Phe Trp Met Val Tyr Tyr Val Met Gly
           35                  40                  45
   Gly Arg Trp Pro Phe Gly Asp Phe Met Cys Arg Ile Trp Met Tyr Phe
       50                  55                  60
   Asp Tyr Met Asn Met Tyr Ala Ser Ile Phe Phe Leu Thr Cys Ile Ser
   65                  70                  75                  80
   Ile Asp Arg Tyr Leu Trp Ala Ile Cys His Pro Met Arg Tyr Met Arg
                   85                  90                  95
   Trp Met Thr Pro Arg His Arg Ala Trp Val Met Ile Ile Ile Ile Trp
               100                 105                 110
   Val Met Ser Phe Leu Ile Ser Met Pro Pro Phe Leu Met Phe Arg Trp
           115                 120                 125
   Ser Thr Tyr Arg Asp Glu Asn Glu Trp Asn Met Thr Trp Cys Met Ile
   130 135 140
   Tyr Asp Trp Pro Glu Trp Met Trp Arg Trp Tyr Val Ile Leu Met Thr
   145 150 155 160
   Ile Ile Met Gly Phe Tyr Ile Pro Met Ile Ile Met Leu Phe Cys Tyr
   165 170 175
   Trp Arg Ile Tyr Arg Ile Ala Arg Leu Trp Met Arg Met Ile Pro Ser
   180 185 190
   Trp Gln Arg Arg Arg Arg Met Ser Met Arg Arg Glu Arg Arg Ile Val
   195 200 205
   Lys Met Leu Ile Ile Ile Met Val Val Phe Ile Ile Cys Trp Leu Pro
   210 215 220
   Tyr Phe Ile Val Met Phe Met Asp Thr Leu Met Met Trp Trp Phe Cys
   225 230 235 240
   Glu Phe Cys Ile Trp Arg Arg Leu Trp Met Tyr Ile Phe Glu Trp Leu
   245 250 255
   Ala Tyr Val Asn Cys Pro Cys Ile Asn Pro Ile Ile Tyr
   260 265

## Description

### FIELD OF THE INVENTION

The present invention relates to a newly identified receptor belonging to the superfamily of G-protein-coupled receptors. The invention also relates to polynucleotides encoding the receptor. The invention further relates to methods using the receptor polypeptides and polynucleotides as a target for diagnosis and treatment in receptor-mediated disorders. The invention further relates to drug-screening methods using the receptor polypeptides and polynucleotides to identify agonists and antagonists for diagnosis and treatment. The invention further encompasses agonists and antagonists based on the receptor polypeptides and polynucleotides. The invention further relates to procedures for producing the receptor polypeptides and polynucleotides.

### BACKGROUND OF THE INVENTION

### G-protein coupled receptors

G-protein coupled receptors (GPCRs) constitute a major class of proteins responsible for transducing a signal within a cell. GPCRs have three structural domains: an amino terminal extracellular domain, a transmembrane domain containing seven transmembrane segments, three extracellular loops, and three intracellular loops, and a carboxy terminal intracellular domain. Upon binding of a ligand to an extracellular portion of a GPCR, a signal is transduced within the cell that results in a change in a biological or physiological property of the cell. GPCRs, along with G-proteins and effectors (intracellular enzymes and channels modulated by G-proteins), are the components of a modular signaling system that connects the state of intracellular second messengers to extracellular inputs.

GPCR genes and gene-products are potential causative agents of disease (Spiegel *et al., J. Clin. Invest. 92*:1119-1125 (1993); McKusick *et al., J. Med Genet. 30*:1-26 (1993)). Specific defects in the rhodopsin gene and the V2 vasopressin receptor gene have been shown to cause various forms of retinitis pigmentosum (Nathans *et al., Annu. Rev. Genet 26*:403-424(1992)), and nephrogenic diabetes insipidus (Holtzman *et al., Hum. Mol. Genet. 2*:1201-1204 (1993)). These receptors are of critical importance to both the central nervous system and peripheral physiological processes. Evolutionary analyses suggest that the ancestor of these proteins originally developed in concert with complex body plans and nervous systems.

The GPCR protein superfamily can be divided into five families: Family I, receptors typified by rhodopsin and the β2-adrenergic receptor and currently represented by over 200 unique members (Dohlman *et al., Annu Rev. Biochem. 60*:653-688 (1991)); Family II, the parathyroid hormone/calcitonin/secretin receptor family (Juppner *et al., Science 254*:1024-1026 (1991); Lin *et al., Science 254*:1022-1024 (1991)); Family III, the metabotropic glutamate receptor family (Nakanishi, *Science 258* 597:603 (1992)); Family IV, the cAMP receptor family, important in the chemotaxis and development of *D. discoideum* (Klein *et al., Science 241*:1467-1472 (1988)); and Family V, the fungal mating pheromone receptors such as STE2 (Kurjan, *Annu. Rev. Biochem. 61*:1097-1129 (1992)).

There are also a small number of other proteins which present seven putative hydrophobic segments and appear to be unrelated to GPCRs; they have not been shown to couple to G-proteins. *Drosophila* expresses a photoreceptor-specific protein, bride of sevenless (boss), a seven-transmembrane-segment protein which has been extensively studied and does not show evidence of being a GPCR (Hart *et al., Proc. Natl. Acad Sci. USA 90*:5047-5051 (1993)). The gene *frizzled (fz)* in *Drosophila* is also thought to be a protein with seven transmembrane segments. Like boss, fz has not been shown to couple to G-proteins (Vinson *et al., Nature 338*:263-264 (1989)).

G proteins represent a family of heterotrimeric proteins composed of α, β and γ subunits, that bind guanine nucleotides. These proteins are usually linked to cell surface receptors, e.g., receptors containing seven transmembrane segments. Following ligand binding to the GPCR, a conformational change is transmitted to the G protein, which causes the α-subunit to exchange a bound GDP molecule for a GTP molecule and to dissociate from the βγ-subunits. The GTP-bound form of the α-subunit typically functions as an effector-modulating moiety, leading to the production of second messengers, such as cAMP (e.g:, by activation of adenyl cyclase), diacylglycerol or inositol phosphates. Greater than 20 different types of α-subunits are known in humans. These subunits associate with a smaller pool of β and γ subunits. Examples of mammalian G proteins include Gi, Go, Gq, Gs and Gt. G proteins are described extensively in Lodish *et al., Molecular Cell Biology,* (Scientific American Books Inc., New York, N.Y.. 1995). GPCRs, G proteins and G protein-linked effector and second messenger systems have been reviewed in *The G-Protein Linked Receptor Fact Book,* Watson *et al.,* eds., Academic Press (1994).

GPCRs are a major target for drug action and development Accordingly, it is valuable to the field of pharmaceutical development to identify and characterize previously unknown GPCRs. The present invention advances the state of the art by providing a previously unidentified human GPCR

### SUMMARY OF THE INVENTION

It is an object of the invention to identify novel GPCRs.

It is a further object of the invention to provide novel GPCR polypeptides that are useful as reagents or targets in receptor assays applicable to treatment and diagnosis of GPCR-mediated disorders.

It is a further object of the invention to provide polynucleotides corresponding to the novel GPCR receptor polypeptides that are useful as targets and reagents in receptor assays applicable to treatment and diagnosis of GPCR-mediated disorders and useful for producing novel receptor polypeptides by recombinant methods.

A specific object of the invention is to identify compounds that act as agonists and antagonists and modulate the expression of the novel receptor.

A further specific object of the invention is to provide compounds that modulate expression of the receptor for treatment and diagnosis of GPCR- related disorders.

The invention is thus based on the identification of a novel GPCR, designated the 15625 receptor.

The invention provides a method for identifying an agent that modulates the level or activity of a polypeptide in a cell or that interacts with a polypeptide in a cell, wherein said polypeptide is selected from:
(a) the amino acid sequence shown in SEQ ID NO: 1 or 3;
(b) the amino acid sequence of an alleic variant of the amino acid sequence shown in SEQ ID NO: 1 or 3;
(c) the amino acid sequence of a sequence variant of the amino acid sequence shown in SEQ ID NO: 1 or 3, wherein the sequence variant is encoded by a nucleic acid molecule hybridizing to the nucleic acid molecule shown in SEQ ID NO: 2 or 4, respectively, under stringent conditions;
(d) a fragment of the amino acid sequence shown in SEQ ID NO: 1 or 3, wherein the fragment comprises at least 10 contiguous amino acids;
(e) the amino acid sequence of the mature receptor polypeptide from about amino acid 6 to about amino acid 342, shown in SEQ ID NO: 1 or 3;
(f) the amino acid sequence of the polypeptide shown in SEQ ID NO: 1 or 3, from about amino acid 1 to about amino acid 342; and
(g) the amino acid sequence of an epitope bearing region of any one of the polypeptides of (a)-(f);
   and said method comprises:
   (i) contacting said agent with a cell capable of expressing said polypeptide such that said polypeptide level or activity can be modulated in said cell by said agent, wherein said cell is derived from brain, CD34⁺ cells, B cells, skeletal muscle, lymph node, spleen, thymus, liver, tonsil, colon, heart, granulocytes, erythroblasts or pancreas; and
   (ii) measuring said polypeptide level or activity.

The invention also provides a method for detecting the presence of a polypeptide in a sample, said method comprising;
(i) contacting said sample with an antibody that specifically allows detection of the presence of the polypeptide in the sample; and
(ii) detecting the presence of the polypeptide, wherein said polypeptide is selected from:
   (a) the amino acid sequence shown in SEQ ID NO: 1 or 3;
   (b) the amino acid sequence of an alleic variant of the amino acid sequence shown in SEQ ID NO: 1 or 3;
   (c) the amino acid sequence of a sequence variant of the amino acid sequence shown in SEQ ID NO: 1 or 3, wherein the sequence variant is encoded by a nucleic acid molecule hybridizing to the nucleic acid molecule shown in SEQ ID NO: 2 or 4, respectively, under stringent conditions;
   (d) a fragment of the amino acid sequence shown in SEQ ID NO: 1 or 3, wherein the fragment comprises at least 10 contiguous amino acids;
   (e) the amino acid sequence of the mature receptor polypeptide from about amino acid 6 to about amino acid 342, shown in SEQ ID NO: 1 or 3;
   (f) the amino acid sequence of the polypeptide shown in SEQ ID NO: 1 or 3, from about amino acid 1 to about amino acid 342;
   (g) the amino acid sequence of an epitope bearing region of any one of the polypeptides of (a)-(f);
   and wherein said sample is derived from a cell selected from brain, CD34⁺, B, skeletal muscle, lymph node, spleen, thymus, liver, tonsil, colon, heart, granulocyte, erthroblast and pancreatic cells.

The invention also provides a method for identifying an agent that modulates the level or activity of a nucleic acid molecule in a cell or that interacts with a nucleic acid molecule in a cell, wherein said nucleic acid molecule has a nucleic acid sequence selected from:
(a) the nucleotide sequence shown in SEQ ID NO: 2 or 4;
(b) a nucleotide sequence encoding the amino sequence shown in SEQ ID NO: 1 or 3;
(c) a nucleotide sequence complementary to any of the nucleotide sequences in (a) or (b);
(d) a nucleotide sequence encoding an amino acid sequence of a sequence variant of the amino acid sequence shown in SEQ ID NO: 1 or 3 that hybridizes to the nucleotide sequence shown in SEQ ID NO: 2 or 4, respectively, under stringent conditions;
(e) a nucleotide sequence complementary to the nucleotide sequence in (d);
(f) a nucleotide sequence encoding a fragment of the amino acid sequence shown in SEQ ID NO: 1 or 3, wherein the fragment comprises at least 10 contiguous amino acids; and
(g) a nucleotide sequence complementary to the nucleotide sequence in (f);
   and said method comprises:
   (i) contacting said agent with a cell capable of expressing said nucleic acid molecule such that said nucleic acid molecule level or activity can be modulated in said cell by said agent, wherein said cell is derived from brain, CD34⁺ cells, B cells, skeletal muscle, lymph node, spleen, thymus, liver, tonsil, colon, heart, granulocytes, erythroblasts or pancreas and;
   (ii) measuring said nucleic acid molecule level or activity.

The invention also provides a method for detecting the presence of a nucleic acid molecule in a sample, said method comprising:
(i) contacting said sample with an agent that specifically allows detection of the presence of the nucleic acid molecule in the sample and;
(ii) detecting the presence of the nucleic acid molecule or fragment thereof, wherein the nucleic acid molecule has a nucleotide sequence selected from:
   (a) the nucleotide sequence shown in SEQ ID NO: 2 or 4;
   (b) a nucleotide sequence encoding the amino acid sequence shown in SEQ ID NO: 1 or 3;
   (c) a nucleotide sequence complementary to any of the nucleotide sequences in (a) or (b);
   (d) a nucleotide sequence encoding an amino acid sequence of a sequence variant of the amino acid sequence shown in SEQ ID NO: 1 or 3 that hybridizes to the nucleotide sequence shown in SEQ ID NO: 2 or 4, respectively, under stringent conditions;
   (e) a nucleotide sequence complementary to the nucleotide sequence in (d);
   (f) a nucleotide sequence encoding a fragment of the amino acid sequence shown in SEQ ID NO: 1 or 3, wherein the fragment comprises at least 10 contiguous amino acids; and
   (g) a nucleotide sequence complementary to the nucleotide sequence in (f);
   wherein said sample is derived from a tissue selected from brain, CD34⁺ cells, B cells, skeletal muscle, lymph node, spleen, thymus, liver, tonsil, colon, heart, granulocytes, erythroblasts and pancreas, and wherein said agent comprises an oligonucleotide that hybridizes to any one of the nucleic acid sequences of (a)-(g) under stringent conditions and step (ii) comprises determining whether the oligonucleotide binds to the nucleic acid sequence in the sample.

### DESCRIPTION OF THE DRAWINGS

**Figure 1** shows an analysis of the 15625 amino acid sequence: αβturn and coil regions; hydrophilicity; amphipathic regions; flexible regions; antigenic index; and surface probability plot.
**Figure 2** shows a 15625 receptor hydrophobicity plot. The amino acids correspond to 1-342 and show the seven transmembrane segments.

### DETAILED DESCRIPTION OF THE INVENTION

### Receptor function/signal pathway

The 15625 receptor protein is a GPCR that participates in signaling pathways. As used herein, a "signaling pathway" refers to the modulation (e.g., stimulation or inhibition) of a cellular function/activity upon the binding of a ligand to the GPCR (15625 protein). Examples of such functions include mobilization of intracellular molecules that participate in a signal transduction pathway, e.g., phosphatidylinositol 4,5-bisphosphate (PIP₂), inositol 1,4,5-triphosphate (IP₃) and adenylate cyclase; polarization of the plasma membrane; production or secretion of molecules; alteration in the structure of a cellular component; cell proliferation, e.g., synthesis ofDNA; cell migration; cell differentiation; and cell survival. The 15625 receptor protein is expressed throughout the brain, primarily in glial cells. It is also highly expressed in bone marrow CD34⁺ cells including, but not limited to, megakaryocytes. It is also moderately expressed in resting B lymphocytes, the level decreasing when these lymphocytes are activated, and in skeletal muscle. The gene is also expressed in lymph node, spleen, thymus, liver, tonsils, colon, heart, granulocytes and erythroblasts. It is also expressed in placenta, and pancreas. Accordingly, cells participating in a 15625 receptor protein signaling pathway include, but are not limited to cells derived from these tissues.

The response mediated by the receptor protein depends on the type of cell. For example, in some cells, binding of a ligand to the receptor protein may stimulate an activity such as release of compounds, gating of a channel, cellular adhesion, migration, differentiation, etc., through phosphatidylinositol or cyclic AMP metabolism and turnover while in other cells, the binding of the ligand will produce a different result. Regardless of the cellular activity/response modulated by the receptor protein, it is universal that the protein is a GPCR and interacts with G proteins to produce one or more secondary signals, in a variety of intracellular signal transduction pathways, e.g., through phosphatidylinositol or cyclic AMP metabolism and turnover, in a cell.

As used herein, "phosphatidylinositol turnover and metabolism" refers to the molecules involved in the turnover and metabolism of phosphatidylinositol 4,5-bisphosphate (PEP₂) as well as to the activities of these molecules. PIP₂ is a phospholipid found in the cytosolic leaflet of the plasma membrane. Binding of ligand to the receptor activates, in some cells, the plasma-membrane enzyme phospholipase C that in turn can hydrolyze PIP₂ to produce 1,2-diacylglycerol (DAG) and inositol 1,4,5-triphosphate (IP₃). Once formed EP₃ can diffuse to the endoplasmic reticulum surface where it can bind an IP₃ receptor, e.g., a calcium channel protein containing an IP₃ binding site. IP₃ binding can induce opening of the channel, allowing calcium ions to be released into the cytoplasm. IP₃ can also be phosphorylated by a specific kinase to form inositol 1,3,4,5-tetraphosphate (IP₄), a molecule which can cause calcium entry into the cytoplasm from the extracellular medium. IP₃ and IP₄ can subsequently be hydrolyzed very rapidly to the inactive products inositol 1,4-biphosphate (IP₂) and inositol 1,3,4-triphosphate, respectively. These inactive products can be recycled by the cell to synthesize PIP₂. The other second messenger produced by the hydrolysis of PIP₂, namely 1,2-diacylglycerol (DAG), remains in the cell membrane where it can serve to activate the enzyme protein kinase C. Protein kinase C is usually found soluble in the cytoplasm of the cell, but upon an increase in the intracellular calcium concentration, this enzyme can move to the plasma membrane where it can be activated by DAG. The activation of protein kinase C in different cells results in various cellular responses such as the phosphorylation of glycogen synthase, or the phosphorylation of various transcription factors, e.g., NF-kB. The language "phosphatidylinositol activity", as used herein, refers to an activity of PIP₂ or one of its metabolites.

Another signaling pathway in which the receptor may participate is the cAMP turnover pathway. As used herein, "cyclic AMP turnover and metabolism" refers to the molecules involved in the turnover and metabolism of cyclic AMP (cAMP) as well as to the activities of these molecules. Cyclic AMP is a second messenger produced in response to ligand-induced stimulation of certain G protein coupled receptors. In the cAMP signaling pathway, binding of a ligand to a GPCR can lead to the activation of the enzyme adenyl cyclase, which catalyzes the synthesis of cAMP. The newly synthesized cAMP can in turn activate a cAMP-dependent protein kinase. This activated kinase can phosphorylate a voltage-gated potassium channel protein, or an associated protein, and lead to the inability of the potassium channel to open during an action potential. The inability of the potassium channel to open results in a decrease in the outward flow of potassium, which normally repolarizes the membrane of a neuron, leading to prolonged membrane depolarization.

### Polypeptides

The invention is based on the discovery of a novel G-coupled protein receptor. Specifically, an expressed sequence tag (EST) was selected based on homology to G-protein-coupled receptor sequences. This EST was used to design primers based on sequences that it contains and used to identify a cDNA from a human cDNA library. Positive clones were sequenced and the overlapping fragments were assembled. Analysis of the assembled sequence revealed that the cloned cDNA molecule encodes a G-protein coupled receptor.

A cDNA nucleotide sequence (SEQ ID NO 4) and the deduced amino acid sequence (SEQ ID NO 3) for a nonhuman primate (macaque brain), corresponding to the human 15625 receptor amino acid and nucleotide sequence is also encompassed by the invention.

The invention thus relates to a novel GPCR having the deduced amino acid sequence shown in (SEQ ID NO 1).

It is predicted that amino acids 1-25 constitute the amino terminal extracellular domain, amino acids 26-302 constitute the region spanning the transmembrane domain, and amino acids 303-342 constitute the carboxy terminal intracellular domain. The transmembrane domain contains seven transmembrane segments, three extracellular loops and three intracellular loops. The transmembrane segments are found from about amino acid 26 to about amino acid 47, from about amino acid 59 to about amino acid 79, from about amino acid 99 to about amino acid 120, from about amino acid 143 to about amino acid 162, from about amino acid 189 to about amino acid 212, from about amino acid 238 to about amino acid 255, and from about amino acid 284 to about amino acid 302. Within the region spanning the entire transmembrane domain are three intracellular and three extracellular loops. The three intracellular loops are found from about amino acid 48 to about amino acid 58, from about amino acid 121 to about amino acid 142, and from about amino acid 213 to about amino acid 237. The three extracellular loops are found at from about amino acid 80 to about amino acid 98, from about amino acid 163 to about amino acid 188, and from about amino acid 256 to about amino acid 283.

The transmembrane domain includes a GPCR signal transduction signature, DRY, at residues 121-123. The sequence includes an arginine at residue 122, an invariant amino acid in GPCRs.

A comparison of the 15625 receptor against the Prosite data base of protein patterns shows a high score against the seven transmembrane segment rhodopsin superfamily. The underlined area shows a GPCR signature, and specifically the position of an arginine residue, conserved in GPCRs. The most commonly conserved sequence is an aspartate, arginine, tyrosine (DRY) triplet. DRY is implicated in signal transduction. Arginine is invariant. Aspartate is conservatively placed in several GPCRs. In the present case, the arginine is found in the sequence DRY, which matches the position of DRY or invariant arginine in GPCRs of the rhodopsin superfamily of receptors.

An analysis of the 15625 open reading frame shows amino acids corresponding to specific functional sites. An N-glycosylation site is found at about amino acids 6-9 and 13-16. A cAMP and cGMP-dependent protein kinase phosphorylation site is found at about amino acids 173-176. A protein kinase C phosphorylation site is found at about amino acids 126-128, 163-165, and 304-306. An N-myristoylation site is found at about amino acids 39-44 and 333-338. In addition, amino acids corresponding in position to the GPCR signature and containing the invariant arginine are found in the sequence DRY at amino acids 121-123.

The "15625 receptor polypeptide" or "15625 receptor protein" refers to the polypeptide in SEQ ID NO 1. The term "receptor protein" or "receptor polypeptide", however, further includes the numerous variants described herein, as well as fragments derived from the full length 15625 polypeptide and variants, for example, SEQ ID NO 3.

The present invention thus provides an isolated or purified 15625 receptor polypeptide and variants and fragments thereof.

The 15625 polypeptide is a 342 residue protein exhibiting three main structural domains. The amino terminal extracellular domain is identified to be within residues 1 to about 25 in SEQ ID NO 1. The transmembrane domain is identified to be within residues from about 26 to about 302 in SEQ ID NO 1. The carboxy terminal intracellular domain is identified to be within residues from about 303 to 342 in SEQ ID NO 1. The transmembrane domain contains seven segments that span the membrane. The transmembrane segments are found from about amino acid 26 to about amino acid 47, from about amino acid 59 to about amino acid 79 from about amino acid 99 to about amino acid 120, from about amino acid 143 to about amino acid 162, from about amino acid 189 to about amino acid 212, from about amino acid 238 to about amino acid 255, and from about amino acid 284 to about amino acid 302. Within the region spanning the entire transmembrane domain are three intracellular and three extracellular loops. The three intracellular loops are found from about amino acid 48 to about amino acid 58, from about amino acid 121 to about amino acid 142, and from about amino acid 213 to about amino acid 237. The three extracellular loops are found at from about amino acid 80 to about amino acid 98, from about amino acid 163 to about amino acid 188, and from about amino acid 256 to about amino acid 283.

The transmembrane domain includes a GPCR signal transduction signature, DRY, at residues 121-123. The sequence includes an arginine at residue 122, an invariant amino acid in GPCRs.

Based on a BLAST search, highest homology was shown to the rhodopsin superfamily of GPCRs.

As used herein, a polypeptide is said to be "isolated" or "purified" when it is substantially free of cellular material when it is isolated from recombinant and non-recombinant cells, or free of chemical precursors or other chemicals when it is chemically synthesized. A polypeptide, however, can be joined to another polypeptide with which it is not normally associated in a cell and still be considered "isolated" or "purified."

The receptor polypeptides can be purified to homogeneity. It is understood, however, that preparations in which the polypeptide is not purified to homogeneity are useful and considered to contain an isolated form of the polypeptide. The critical feature is that the preparation allows for the desired function of the polypeptide, even in the presence of considerable amounts of other components. Thus, the invention encompasses various degrees of purity.

In one embodiment, the language "substantially free of cellular material" includes preparations of the receptor polypeptide having less than about 30% (by dry weight) other proteins (i.e., contaminating protein), less than about 20% other proteins, less than about 10% other proteins, or less than about 5% other proteins. When the receptor polypeptide is recombinantly produced, it can also be substantially free of culture medium, i.e., culture medium represents less than about 20%, less than about 10%, or less than about 5% of the volume of the protein preparation.

The language "substantially free of chemical precursors or other chemicals" includes preparations of the receptor polypeptide in which it is separated from chemical precursors or other chemicals that are involved in its synthesis. In one embodiment, the language "substantially free of chemical precursors or other chemicals" includes preparations of the polypeptide having less than about 30% (by dry weight) chemical precursors or other chemicals, less than about 20% chemical precursors or other chemicals, less than about 10% chemical precursors or other chemicals, or less than about 5% chemical precursors or other chemicals.

In one embodiment, the receptor polypeptide comprises the amino acid sequence shown in SEQ ID NO 1. However, the invention also encompasses sequence variants. Variants include a substantially homologous protein encoded by the same genetic locus in an organism, i.e., an allelic variant. The 15625 receptor has been mapped to chromosome 3, in proximity to the AFM 64YG9 marker. Variants also encompass proteins derived from other genetic loci in an organism, but having substantial homology to the 15625 receptor protein of SEQ ID NO 1. Variants also include proteins substantially homologous to the 15625 receptor protein but derived from another organism, i.e., an ortholog, such as in SEQ ID NO 3. Variants also include proteins that are substantially homologous to the 15625 receptor protein that are produced by chemical synthesis. Variants also include proteins that are substantially homologous to the 15625 receptor protein that are produced by recombinant methods. It is understood, however, that variants exclude any amino acid sequences disclosed prior to the invention.

As used herein, two proteins (or a region of the proteins) are substantially homologous when the amino acid sequences are at least about 50-55%, 55-60%, typically at least about 70-75%, more typically at least about 80-85%, and most typically at least about 90-95% or more homologous. A substantially homologous amino acid sequence, according to the present invention, will be encoded by a nucleic acid sequence hybridizing to the nucleic acid sequence, or portion thereof, of the sequence shown in SEQ ID NO 2 under stringent conditions as more fully described below.

To determine the percent identity of two amino acid sequences or of two nucleic acid sequences, the sequences are aligned for optimal comparison purposes (e.g., gaps can be introduced in one or both of a first and a second amino acid or nucleic acid sequence for optimal alignment and non-homologous sequences can be disregarded for comparison purposes). In a preferred embodiment, the length of a reference sequence aligned for comparison purposes is at least 30%, preferably at least 40%, more preferably at least 50%, even more preferably at least 60%, and even more preferably at least 70%, 80%, or 90% of the length of the reference sequence (e.g., when aligning a second sequence to the amino acid sequences herein, having 342 amino acid residues, at least 80, preferably at least 100, more preferably at least 120, even more preferably at least 140, and even more preferably at least 200, 250, 300, or 340 amino acid residues are aligned). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology"). The percent identity between the two sequences is a function of the number of identical positions shared by the sequences, taking into account the number of gaps, and the length of each gap, which need to be introduced for optimal alignment of the two sequences.

The invention also encompasses polypeptides having a lower degree of identity but having sufficient similarity so as to perform one or more of the same functions performed by the 15625 polypeptide. Similarity is determined by conserved amino acid substitution. Such substitutions are those that substitute a given amino acid in a polypeptide by another amino acid of like characteristics. Conservative substitutions are likely to be phenotypically silent. Typically seen as conservative substitutions are the replacements, one for another, among the aliphatic amino acids Ala, Val, Leu, and Ile; interchange of the hydroxyl residues Ser and Thr, exchange of the acidic residues Asp and Glu, substitution between the amide residues Asn and Gln, exchange of the basic residues Lys and Arg and replacements among the aromatic residues Phe, Tyr. Guidance concerning which amino acid changes are likely to be phenotypically silent are found in Bowie *et al., Science 247*:1306-1310 (1990).

**TABLE 1. Conservative Amino Acid Substitutions.**

| | |
|---|---|
| Aromatic | Phenylalanine |
| | Tryptophan |
| | Tyrosine |
| Hydrophobic | Leucine |
| | Isoleucine |
| | Valine |
| Polar | Glutamine |
| | Asparagine |
| Basic | Arginine |
| | Lysine |
| | Histidine |
| Acidic | Aspartic Acid |
| | Glutamic Acid |
| Small | Alanine |
| | Serine |
| | Threonine |
| | Methionine |
| | Glycine |

The comparison of sequences and determination of percent identity and similarity between two sequences can be accomplished using a mathematical algorithm. (*Computational Molecular Biology,* Lesk, A.M., ed., Oxford University Press, New York, 1988; *Biocomputing: Informatics and Genome Projects,* Smith, D.W., ed., Academic Press, New York, 1993; *Computer Analysis of Sequence Data, Part I,* Griffin, A.M., and Griffin, H.G., eds., Humana Press, New Jersey, 1994; *Sequence Analysis in Molecular Biology,* von Heinje, G., Academic Press, 1987; and *Sequence Analysis Primer,* Gribskov, M. and Devereux, J., eds., M Stockton Press, New York, 1991). In a preferred embodiment, the percent identity between two amino acid sequences is determined using the Needleman and Wunsch (*J. Mol. Biol. (48)*:444-453 (1970)) algorithm which has been incorporated into the GAP program in the GCG software package (available at http://www.gcg.com), using either a Blossom 62 matrix or a PAM250 matrix, and a gap weight of 16, 14, 12, 10, 8, 6, or 4 and a length weight of 1, 2, 3, 4, 5, or 6. In yet another preferred embodiment, the percent identity between two nucleotide sequences is determined using the GAP program in the GCG software package (Devereux, J., *et al., Nucleic Acids Res. 12(I)*:387 (1984)) (available at http://www.gcg.com), using a NWSgapdna.CMP matrix and a gap weight of 40, 50, 60, 70, or 80 and a length weight of 1, 2, 3, 4, 5, or 6. In another embodiment, the percent identity between two amino acid or nucleotide sequences is determined using the algorithm ofE. Meyers and W. Miller (CABIOS, 4:11-17 (1989)) which has been incorporated into the ALIGN program (version 2.0), using a PAM120 weight residue table, a gap length penalty of 12 and a gap penalty of 4.

The nucleic acid and protein sequences of the present invention can further be used as a "query sequence" to perform a search against public databases to, for example, identify other family members or related sequences. Such searches can be performed using the NBLAST and XBLAST programs (version 2.0) of Altschul, *et al.* (*J. Mol. Biol.* 215:403-10 (1990)). BLAST nucleotide searches can be performed with the NBLAST program, score = 100, wordlength = 12 to obtain nucleotide sequences homologous to the nucleic acid molecules of the invention. BLAST protein searches can be performed with the XBLAST program, score = 50, wordlength = 3 to obtain amino acid sequences homologous to the proteins of the invention. To obtain gapped alignments for comparison purposes, Gapped BLAST can be utilized as described in Altschul et al. (*Nucleic Acids Res.* 25(17):3389-3402 (1997)). When utilizing BLAST and gapped BLAST programs, the default parameters of the respective programs (e.g., XBLAST and NBLAST) can be used. See http://www.ncbi.nlm.nih.gov.

A variant polypeptide can differ in amino acid sequence by one or more substitutions, deletions, insertions, inversions, fusions, and truncations or a combination of any of these.

Variant polypeptides can be fully functional or can lack function in one or more activities. Thus, in the present case, variations can affect the function, for example, of one or more of the regions corresponding to ligand binding, membrane association, G-protein binding and signal transduction.

Fully functional variants typically contain only conservative variation or variation in non-critical residues or in non-critical regions. Functional variants can also contain substitution of similar amino acids which result in no change or an insignificant change in function. Alternatively, such substitutions may positively or negatively affect function to some degree.

Non-functional variants typically contain one or more non-conservative amino acid substitutions, deletions, insertions, inversions, or truncation or a substitution, insertion, inversion, or deletion in a critical residue or critical region.

As indicated, variants can be naturally-occurring or can be made by recombinant means or chemical synthesis to provide useful and novel characteristics for the receptor polypeptide. This includes preventing immunogenicity from pharmaceutical formulations by preventing protein aggregation.

Useful variations further include alteration of ligand binding characteristics. For example, one embodiment involves a variation at the binding site that results in binding but not release, or slower release, of ligand. A further useful variation at the same sites can result in a higher affinity for ligand. Useful variations also include changes that provide for affinity for another ligand. Another useful variation includes one that allows binding but which prevents activation by the ligand. Another useful variation includes variation in the transmembrane G-protein-binding/signal transduction domain that provides for reduced or increased binding by the appropriate G-protein or for binding by a different G-protein than the one with which the receptor is normally associated. Another useful variation provides a fusion protein in which one or more domains or subregions is operationally fused to one or more domains or subregions from another G-protein coupled receptor.

Amino acids that are essential for function can be identified by methods known in the art, such as site-directed mutagenesis or alanine-scanning mutagenesis (Cunningham *et al., Science 244*:1081-1085 (1989)). The latter procedure introduces single alanine mutations at every residue in the molecule. The resulting mutant molecules are then tested for biological activity such as receptor binding or *in vitro,* or *in vitro* proliferative activity. Sites that are critical for ligand-receptor binding can also be determined by structural analysis such as crystallization, nuclear magnetic resonance or photoaffinity labeling (Smith *et al., J. Mol. Biol. 224*:899-904 (1992); de Vos *et al. Science 255*:306-312 (1992)).

Substantial homology can be to the entire nucleic acid or amino acid sequence or to fragments of these sequences.

The invention thus also includes polypeptide fragments of the 15625 receptor protein. Fragments can be derived from the amino acid sequence shown in SEQ ID NO 1. However, the invention also encompasses fragments of the variants of the 15625 receptor protein as described herein.

The fragments to which the invention pertains, however, are not to be construed as encompassing fragments that may be disclosed prior to the present invention.

As used herein, a fragment comprises at least 10 contiguous amino acids from amino acid 1 to amino acid 280 and from amino acid 291 to amino acid 342. Fragments retain one or more of the biological activities of the protein, for example the ability to bind to a G-protein or ligand, as well as fragments that can be used as an immunogen to generate receptor antibodies.

Biologically active fragments (peptides which are, for example, 10, 12, 15, 20, 30, 35, 36, 37, 38, 39, 40, 50, 100 or more amino acids in length) can comprise a domain or motif, e.g., an extracellular or intracellular domain or loop, one or more transmembrane segments, or parts thereof, G-protein binding site, or GPCR signature, glycosylation sites, cAMP and cGMP-dependent protein kinase and protein kinase C phosphorylation sites, and myristoylation sites.

Possible fragments include, but are not limited to: 1) soluble peptides comprising the entire amino terminal extracellular domain about amino acid 1 to about amino acid 25 of SEQ ID NO 1 or parts thereof; 2) peptides comprising the entire carboxy terminal intracellular domain from about amino acid 303 to amino acid 342 of SEQ ID NO 1 or parts thereof; 3) peptides comprising the region spanning the entire transmembrane domain from about amino acid 26 to amino acid 302; 4) any of the specific transmembrane segments, or parts thereof; 5) any of the three intracellular or three extracellular loops, or parts thereof.

Fragments can retain one or more of the biological activities of the protein, for example the ability to bind to a G-protein or ligand. Fragments can also be useful as an immunogen to generate receptor antibodies.

Biologically active fragments can comprise a domain or motif, e.g., an extracellular or intracellular domain or loop, one or more transmembrane segments, or parts thereof, G-protein binding site, or GPCR signature, glycosylation, cAMP and cGMP-dependent protein kinase phosphorylation sites, protein kinase C phosphorylation sites, and N-myristoylation sites. Such peptides can be, for example, 10, 15, 20, 30, 35, 36, 37, 38, 39, 40, 50, 100 or more amino acids in length.

Possible fragments include, but are not limited to: 1) soluble peptides comprising the entire amino terminal extracellular domain about amino acid 1 to about amino acid 25 of SEQ ID NO 1, or parts thereof; 2) peptides comprising the entire carboxy terminal intracellular domain from about amino acid 303 to amino acid 342 of SEQ ID NO 1, or parts thereof; 3) peptides comprising the region spanning the entire transmembrane domain from about amino acid 26 to about amino acid 302, or parts thereof; 4) any of the specific transmembrane segments, or parts thereof, from about amino acid 26 to about amino acid 47, from about amino acid 59 to about amino acid 79, from about amino acid 99 to about amino acid 120, from about amino acid 143 to about amino acid 162, from about amino acid 189 to about amino acid 212, from about amino acid 238 to about amino acid 255, and from about amino acid 284 to about amino acid 302; 5) any of the three intracellular or three extracellular loops, or parts thereof, from about amino acid 48 to about amino acid 58, from about amino acid 121 to about amino acid 142, from about amino acid 213 to about amino acid 237, from about amino acid 80 to about amino acid 98, from about amino acid 163 to about amino acid 188, and from about amino acid 256 to about amino acid 283. Fragments further include combinations of the above fragments, such as an amino terminal domain combined with one or more transmembrane segments and the attendant extra or intracellular loops or one or more transmembrane segments, and the attendant intra or extracellular loops, plus the carboxy terminal domain. Thus, any of the above fragments can be combined. Other fragments include the mature protein from about amino acid 6 to 342. Other fragments contain the various functional sites described herein, such as glycosylation, cAMP and cGMP-dependent protein kinase phosphorylation sites, protein kinase C phosphorylation sites, N-myristoylation sites, and a sequence containing the GPCR signature sequence. Fragments, for example, can extend in one or both directions from the functional site to encompass 5, 10, 15, 20, 30, 40, 50, or up to 100 amino acids. Further, fragments can include sub-fragments of the specific domains mentioned above, which sub-fragments retain the function of the domain from which they are derived.

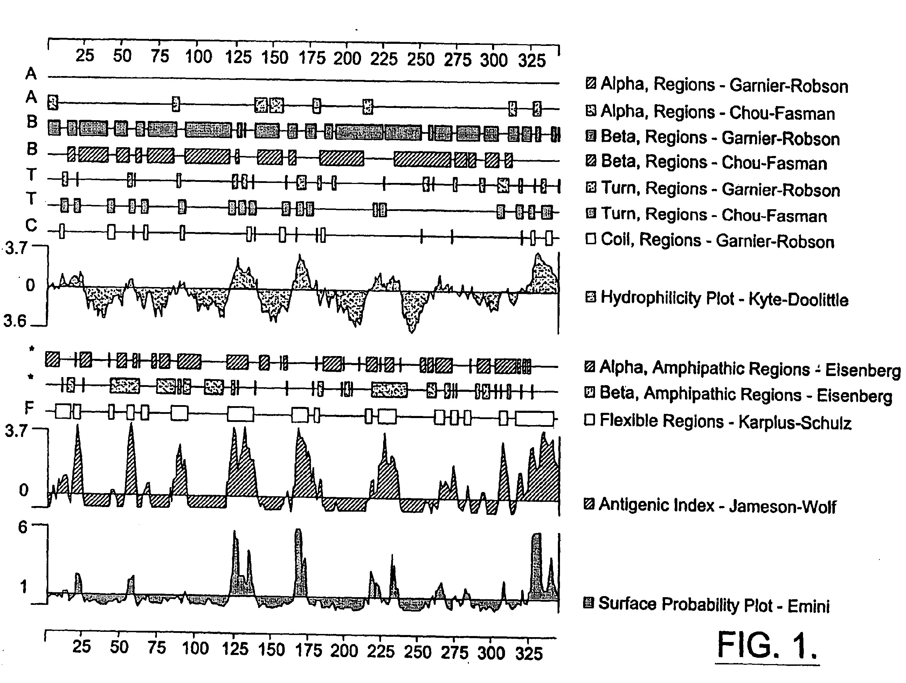

Fragments also include antigenic fragments and specifically those shown to have a high antigenic index in Figure 1.

Accordingly, possible fragments include fragments defining a ligand-binding site, fragments defining membrane association, fragments defining interaction with G proteins and signal transduction, and fragments defining glycosylation sites, cAMP and cGMP-dependent protein kinase phosphorylation sites, protein kinase C phosphorylation sites, and N-myristoylation sites. By this is intended a discrete fragment that provides the relevant function or allows the relevant function to be identified. In a preferred embodiment, the fragment contains the ligand-binding site.

The invention also provides fragments with immunogenic properties. These contain an epitope-bearing portion of the 15625 receptor protein and variants. These epitope-bearing peptides are useful to raise antibodies that bind specifically to a receptor polypeptide or region or fragment. These peptides can contain at least 10, 12, at least 14, or between at least about 15 to about 30 amino acids.

Non-limiting examples of antigenic polypeptides that can be used to generate antibodies include peptides derived from the amino terminal extracellular domain or any of the extracellular loops. Regions having a high antigenicity index are shown in Figure 1. However, intracellularly-made antibodies ("intrabodies") are also encompassed, which would recognize intracellular receptor peptide regions.

The receptor polypeptides (including variants and fragments which may have been disclosed prior to the present invention) are useful for biological assays related to GPCRs. Such assays involve any of the known GPCR functions or activities or properties useful for diagnosis and treatment of GPCR-related conditions.

The epitope-bearing receptor and polypeptides may be produced by any conventional means (Houghten, R.A., *Proc. Natl. Acad Sci. USA 82*:5131-5135 (1985)). Simultaneous multiple peptide synthesis is described in U.S. Patent No. 4,631,211.

Fragments can be discrete (not fused to other amino acids or polypeptides) or can be within a larger polypeptide. Further, several fragments can be comprised within a single larger polypeptide. In one embodiment a fragment designed for expression in a host can have heterologous pre- and pro-polypeptide regions fused to the amino terminus of the receptor fragment and an additional region fused to the carboxyl terminus of the fragment.

The invention thus provides chimeric or fusion proteins. These comprise a receptor protein operatively linked to a heterologous protein having an amino acid sequence not substantially homologous to the receptor protein. "Operatively linked" indicates that the receptor protein and the heterologous protein are fused in-frame. The heterologous protein can be fused to the N-terminus or C-terminus of the receptor protein.

In one embodiment the fusion protein does not affect receptor function *per se.* For example, the fusion protein can be a GST-fusion protein in which the receptor sequences are fused to the C-terminus of the GST sequences. Other types of fusion proteins include, but are not limited to, enzymatic fusion proteins, for example beta-galactosidase fusions, yeast two-hybrid GAL fusions, poly-His fusions and Ig fusions. Such fusion proteins, particularly poly-His fusions, can facilitate the purification of recombinant receptor protein. In certain host cells (e.g., mammalian host cells), expression and/or secretion of a protein can be increased by using a heterologous signal sequence. Therefore, in another embodiment, the fusion protein contains a heterologous signal sequence at its N-terminus.

EP-A-O 464 533 discloses fusion proteins comprising various portions of immunoglobulin constant regions. The Fc is useful in therapy and diagnosis and thus results, for example, in improved pharmacokinetic properties (EP-A 0232 262). In drug discovery, for example, human proteins have been fused with Fc portions for the purpose of high-throughput screening assays to identify antagonists. Bennett *et al.* (*J. Mol. Recog. 8*:52-58 (1995)) and Johanson *et al.* (*J. Biol. Chem. 270. 16*:9459-9471 (1995)). Thus, this invention also encompasses soluble fusion proteins containing a receptor polypeptide and various portions of the constant regions of heavy or light chains of immunoglobulins of various subclass (IgG, IgM, IgA, IgE). Preferred as immunoglobulin is the constant part of the heavy chain of human IgG, particularly IgG1, where fusion takes place at the hinge region. For some uses it is desirable to remove the Fc after the fusion protein has been used for its intended purpose, for example when the fusion protein is to be used as antigen for immunizations. In a particular embodiment, the Fc part can be removed in a simple way by a cleavage sequence which is also incorporated and can be cleaved with factor Xa.

A chimeric or fusion protein can be produced by standard recombinant DNA techniques. For example, DNA fragments coding for the different protein sequences are ligated together in-frame in accordance with conventional techniques. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed and re-amplified to generate a chimeric gene sequence (see Ausubel *et al., Current Protocols in Molecular Biology,* 1992). Moreover, many expression vectors are commercially available that already encode a fusion moiety (e.g., a GST protein). A receptor protein-encoding nucleic acid can be cloned into such an expression vector such that the fusion moiety is linked in-frame to the receptor protein.

Another form of fusion protein is one that directly affects receptor functions. Accordingly, a receptor polypeptide is encompassed by the present invention in which one or more of the receptor domains (or parts thereof) has been replaced by homologous domains (or parts thereof) from another G-protein coupled receptor or other type of receptor. Accordingly, various permutations are possible. The amino terminal extracellular domain, or subregion thereof, (for example, ligand-binding) can be replaced with the domain or subregion from another ligand-binding receptor protein. Alternatively, the entire transmembrane domain, or any of the seven segments or loops, or parts thereof, for example, G-protein-binding/signal transduction, can be replaced. Finally, the carboxy terminal intracellular domain or subregion can be replaced. Thus, chimeric receptors can be formed in which one or more of the native domains or subregions has been replaced.

The isolated receptor protein can be purified from cells that naturally express it, as disclosed herein, such as from brain, and especially glial cells, CD34⁺ cells, and lines such as HEK 293 and Jurkat, purified from cells that have been altered to express it (recombinant), or synthesized using known protein synthesis methods.

In one embodiment, the protein is produced by recombinant DNA techniques. For example, a nucleic acid molecule encoding the receptor polypeptide is cloned into an expression vector, the expression vector introduced into a host cell and the protein expressed in the host cell. The protein can then be isolated from the cells by an appropriate purification scheme using standard protein purification techniques.

Polypeptides often contain amino acids other than the 20 amino acids commonly referred to as the 20 naturally-occurring amino acids. Further, many amino acids, including the terminal amino acids, may be modified by natural processes, such as processing and other post-translational modifications, or by chemical modification techniques well known in the art. Common modifications that occur naturally in polypeptides are described in basic texts, detailed monographs, and the research literature, and they are well known to those of skill in the art.

Accordingly, the polypeptides also encompass derivatives or analogs in which a substituted amino acid residue is not one encoded by the genetic code, in which a substituent group is included, in which the mature polypeptide is fused with another compound, such as a compound to increase the half-life of the polypeptide (for example, polyethylene glycol), or in which the additional amino acids are fused to the mature polypeptide, such as a leader or secretory sequence or a sequence for purification of the mature polypeptide or a pro-protein sequence.

Known modifications include, but are not limited to, acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent crosslinks, formation of cystine, formation of pyroglutamate, formylation, gamma carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination.

Such modifications are well-known to those of skill in the art and have been described in great detail in the scientific literature. Several particularly common modifications, glycosylation, lipid attachment, sulfation, gamma-carboxylation of glutamic acid residues, hydroxylation and ADP-ribosylation, for instance, are described in most basic texts, such as *Proteins - Structure and Molecular Properties,* 2nd Ed., T.E. Creighton, W. H. Freeman and Company, New York (1993). Many detailed reviews are available on this subject, such as by Wold, F., *Posttranslational Covalent Modification of Proteins,* B. C. Johnson, Ed., Academic Press, New York 1-12 (1983); Seifter *et al. (Meth. Enzymol. 182:* 626-646 (1990)) and Rattan *et al. (Ann. N.Y. Acad Sci. 663*:48-62 (1992)).

As is also well known, polypeptides are not always entirely linear. For instance, polypeptides may be branched as a result of ubiquitination, and they may be circular, with or without branching, generally as a result of post-translation events, including natural processing event and events brought about by human manipulation which do not occur naturally. Circular, branched and branched circular polypeptides may be synthesized by non-translational natural processes and by synthetic methods.

Modifications can occur anywhere in a polypeptide, including the peptide backbone, the amino acid side-chains and the amino or carboxyl termini. Blockage of the amino or carboxyl group in a polypeptide, or both, by a covalent modification, is common in naturally-occurring and synthetic polypeptides. For instance, the amino terminal residue of polypeptides made in *E. coli,* prior to proteolytic processing, almost invariably will be N-formylmethionine.

The modifications can be a function of how the protein is made. For recombinant polypeptides, for example, the modifications will be determined by the host cell posttranslational modification capacity and the modification signals in the polypeptide amino acid sequence. Accordingly, when glycosylation is desired, a polypeptide should be expressed in a glycosylating host, generally a eukaryotic cell. Insect cells often carry out the same posttranslational glycosylations as mammalian cells and, for this reason, insect cell expression systems have been developed to efficiently express mammalian proteins having native patterns of glycosylation. Similar considerations apply to other modifications.

The same type of modification may be present in the same or varying degree at several sites in a given polypeptide. Also, a given polypeptide may contain more than one type of modification.

The above disclosure generally applies also to the polypeptide sequence shown in SEQ ID NO 3. Predicted domains and functional sites are readily identifiable by computer programs well-known and readily available to those of skill in the art (e.g., PROSITE analysis).

### Polypeptide uses

The receptor polypeptides are useful for producing antibodies specific for the 15625 receptor protein, regions, or fragments. Regions having a high antigenicity index score are shown in Figure 1.

The receptor polypeptides (including variants and fragments which may have been disclosed prior to the present invention) are useful for biological assays related to GPCRs. Such assays involve any of the known GPCR functions or activities or properties useful for diagnosis and treatment of GPCR-related conditions.

The receptor polypeptides are also useful in drug screening assays, in cell-based or cell-free systems. Cell-based systems can be native, i.e., cells that normally express the receptor protein, as a biopsy or expanded in cell culture. In one embodiment, however, cell-based assays involve recombinant host cells expressing the receptor protein.

The polypeptides can be used to identify compounds that modulate receptor activity. Both 15625 protein and appropriate variants and fragments can be used in high-throughput screens to assay candidate compounds for the ability to bind to the receptor. These compounds can be further screened against a functional receptor to determine the effect of the compound on the receptor activity. Compounds can be identified that activate (agonist) or inactivate (antagonist) the receptor to a desired degree.

The receptor polypeptides can be used to screen a compound for the ability to stimulate or inhibit interaction between the receptor protein and a target molecule that normally interacts with the receptor protein. The target can be ligand or a component of the signal pathway with which the receptor protein normally interacts (for example, a G-protein or other interactor involved in cAMP or phosphatidylinositol turnover and/or adenylate cyclase, or phospholipase C activation). The assay includes the steps of combining the receptor protein with a candidate compound under conditions that allow the receptor protein or fragment to interact with the target molecule, and to detect the formation of a complex between the protein and the target or to detect the biochemical consequence of the interaction with the receptor protein and the target, such as any of the associated effects of signal transduction such as G-protein phosphorylation, cAMP or phosphatidylinositol turnover, and adenylate cyclase or phospholipase C activation.

Candidate compounds include, for example, 1) peptides such as soluble peptides, including Ig-tailed fusion peptides and members of random peptide libraries (see, e.g., Lam *et al., Nature 354*:82-84 (1991); Houghten *et al., Nature 354*:84-86 (1991)) and combinatorial chemistry-derived molecular libraries made ofD- and/or L- configuration amino acids; 2) phosphopeptides (e.g., members of random and partially degenerate, directed phosphopeptide libraries, see, e.g., Songyang *et al., Cell* 72:767-778 (1993)); 3) antibodies (e.g., polyclonal, monoclonal, humanized, anti-idiotypic, chimeric, and single chain antibodies as well as Fab, F(ab')₂, Fab expression library fragments, and epitope-binding fragments of antibodies); and 4) small organic and inorganic molecules (e.g., molecules obtained from combinatorial and natural product libraries).

One candidate compound is a soluble full-length receptor or fragment that competes for ligand binding. Other candidate compounds include mutant receptors or appropriate fragments containing mutations that affect receptor function and thus compete for ligand. Accordingly, a fragment that competes for ligand, for example with a higher affinity, or a fragment that binds ligand but does not allow release, is encompassed by the invention.

The invention provides other end points to identify compounds that modulate (stimulate or inhibit) receptor activity. The assays typically involve an assay of events in the signal transduction pathway that indicate receptor activity. Thus, the expression of genes that are up- or down-regulated in response to the receptor protein dependent signal cascade can be assayed. In one embodiment, the regulatory region of such genes can be operably linked to a marker that is easily detectable, such as luciferase. Alternatively, phosphorylation of the receptor protein, or a receptor protein target, could also be measured.

Any of the biological or biochemical functions mediated by the receptor can be used as an endpoint assay. These include all of the biochemical or biochemical/biological events described herein, and other functions known to those of ordinary skill in the art.

Binding and/or activating compounds can also be screened by using chimeric receptor proteins in which the amino terminal extracellular domain, or parts thereof, the entire transmembrane domain or subregions, such as any of the seven transmembrane segments or any of the intracellular or extracellular loops and the carboxy terminal intracellular domain, or parts thereof, can be replaced by heterologous domains or subregions. For example, a G-protein-binding region can be used that interacts with a different G-protein then that which is recognized by the native receptor. Accordingly, a different set of signal transduction components is available as an end-point assay for activation. Alternatively, the entire transmembrane portion or subregions (such as transmembrane segments or intracellular or extracellular loops) can be replaced with the entire transmembrane portion or subregions specific to a host cell that is different from the host cell from which the amino terminal extracellular domain and/or the G-protein-binding region are derived. This allows for assays to be performed in other than the specific host cell from which the receptor is derived. Alternatively, the amino terminal extracellular domain (and/or other ligand-binding regions) could be replaced by a domain (and/or other binding region) binding a different ligand, thus, providing an assay for test compounds that interact with the heterologous amino terminal extracellular domain (or region) but still cause signal transduction. Finally, activation can be detected by a reporter gene containing an easily detectable coding region operably linked to a transcriptional regulatory sequence that is part of the native signal transduction pathway

The receptor polypeptides are also useful in competition binding assays in methods designed to discover compounds that interact with the receptor. Thus, a compound is exposed to a receptor polypeptide under conditions that allow the compound to bind or to otherwise interact with the polypeptide. Soluble receptor polypeptide is also added to the mixture. If the test compound interacts with the soluble receptor polypeptide, it decreases the amount of complex formed or activity from the receptor target. This type of assay is particularly useful in cases in which compounds are sought that interact with specific regions of the receptor. Thus, the soluble polypeptide that competes with the target receptor region is designed to contain peptide sequences corresponding to the region of interest.

To perform cell free drug screening assays, it is desirable to immobilize either the receptor protein, or fragment, or its target molecule to facilitate separation of complexes from uncomplexed forms of one or both of the proteins, as well as to accommodate automation of the assay.

Techniques for immobilizing proteins on matrices can be used in the drug screening assays. In one embodiment, a fusion protein can be provided which adds a domain that allows the protein to be bound to a matrix. For example, glutathione-S-transferase/15625 fusion proteins can be adsorbed onto glutathione sepharose beads (Sigma Chemical, St. Louis, MO) or glutathione derivatized microtitre plates, which are then combined with the cell lysates (e.g., ³⁵S-labeled) and the candidate compound, and the mixture incubated under conditions conducive to complex formation (e.g., at physiological conditions for salt and pH). Following incubation, the beads are washed to remove any unbound label, and the matrix immobilized and radiolabel determined directly, or in the supernatant after the complexes are dissociated. Alternatively, the complexes can be dissociated from the matrix, separated by SDS-PAGE, and the level of receptor-binding protein found in the bead fraction quantified from the gel using standard electrophoretic techniques. For example, either the polypeptide or its target molecule can be immobilized utilizing conjugation of biotin and streptavidin using techniques well known in the art. Alternatively, antibodies reactive with the protein but which do not interfere with binding of the protein to its target molecule can be derivatized to the wells of the plate, and the protein trapped in the wells by antibody conjugation. Preparations of a receptor-binding protein and a candidate compound are incubated in the receptor protein-presenting wells and the amount of complex trapped in the well can be quantitated. Methods for detecting such complexes, in addition to those described above for the GST-immobilized complexes, include immunodetection of complexes using antibodies reactive with the receptor protein target molecule, or which are reactive with receptor protein and compete with the target molecule, as well as enzyme-linked assays which rely on detecting an enzymatic activity associated with the target molecule.

Modulators of receptor protein activity identified according to these drug screening assays can be used to treat a subject with a disorder mediated by the receptor pathway, by treating cells that express the 15625 protein, such as in brain, especially glial cells, bone marrow CD34⁺ cells, including but not limited to, megakaryocytes, resting B lymphocytes, and skeletal muscle. Other tissues include lymph node, spleen, heart, thymus, liver, tonsils, colon, granulocytes, placenta, pancreas, and erythroblasts. These methods of treatment include the steps of administering the modulators of protein activity in a pharmaceutical composition as described herein, to a subject in need of such treatment.

Disorders involving the spleen include, but are not limited to, splenomegaly, including nonspecific acute splenitis, congestive spenomegaly, and spenic infarcts; neoplasms, congenital anomalies, and rupture. Disorders associated with splenomegaly include infections, such as nonspecific splenitis, infectious mononucleosis, tuberculosis, typhoid fever, brucellosis, cytomegalovirus, syphilis, malaria, histoplasmosis, toxoplasmosis, kala-azar, trypanosomiasis, schistosomiasis, leishmaniasis, and echinococcosis; congestive states related to partial hypertension, such as cirrhosis of the liver, portal or splenic vein thrombosis, and cardiac failure; lymphohematogenous disorders, such as Hodgkin disease, non-Hodgkin lymphomas/leukemia, multiple myeloma, myeloproliferative disorders, hemolytic anemias, and thrombocytopenic purpura; immunologic-inflammatory conditions, such as rheumatoid arthritis and systemic lupus erythematosus; storage diseases such as Gaucher disease, Niemann-Pick disease, and mucopolysaccharidoses; and other conditions, such as amyloidosis, primary neoplasms and cysts, and secondary neoplasms.

Disorders involving the colon include, but are not limited to, congenital anomalies, such as atresia and stenosis, Meckel diverticulum, congenital aganglionic megacolon-Hirschsprung disease; enterocolitis, such as diarrhea and dysentery, infectious enterocolitis, including viral gastroenteritis, bacterial enterocolitis, necrotizing enterocolitis, antibiotic-associated colitis (pseudomembranous colitis), and collagenous and lymphocytic colitis, miscellaneous intestinal inflammatory disorders, including parasites and protozoa, acquired immunodeficiency syndrome, transplantation, drug-induced intestinal injury, radiation enterocolitis, neutropenic colitis (typhlitis), and diversion colitis; idiopathic inflammatory bowel disease, such as Crohn disease and ulcerative colitis; tumors of the colon, such as non-neoplastic polyps, adenomas, familial syndromes, colorectal carcinogenesis, colorectal carcinoma, and carcinoid tumors.

Disorders involving the liver include, but are not limited to, hepatic injury; jaundice and cholestasis, such as bilirubin and bile formation; hepatic failure and cirrhosis, such as cirrhosis, portal hypertension, including ascites, portosystemic shunts, and splenomegaly; infectious disorders, such as viral hepatitis, including hepatitis A-E infection and infection by other hepatitis viruses, clinicopathologic syndromes, such as the carrier state, asymptomatic infection, acute viral hepatitis, chronic viral hepatitis, and fulminant hepatitis; autoimmune hepatitis; drug- and toxin-induced liver disease, such as alcoholic liver disease; inborn errors of metabolism and pediatric liver disease, such as hemochromatosis, Wilson disease, *a*_{*1*}-antitrypsin deficiency, and neonatal hepatitis; intrahepatic biliary tract disease, such as secondary biliary cirrhosis, primary biliary cirrhosis, primary sclerosing cholangitis, and anomalies of the biliary tree; circulatory disorders, such as impaired blood flow into the liver, including hepatic artery compromise and portal vein obstruction and thrombosis, impaired blood flow through the liver, including passive congestion and centrilobular necrosis and peliosis hepatis, hepatic vein outflow obstruction, including hepatic vein thrombosis (Budd-Chiari syndrome) and veno-occlusive disease; hepatic disease associated with pregnancy, such as preeclampsia and eclampsia, acute fatty liver of pregnancy, and intrehepatic cholestasis of pregnancy; hepatic complications of organ or bone marrow transplantation, such as drug toxicity after bone marrow transplantation, graft-versus-host disease and liver rejection, and nonimmunologic damage to liver allografts; tumors and tumorous conditions, such as nodular hyperplasias, adenomas, and malignant tumors, including primary carcinoma of the liver and metastatic tumors.

Disorders involving the brain include, but are limited to, disorders involving neurons, and disorders involving glia, such as astrocytes, oligodendrocytes, ependymal cells, and microglia; cerebral edema, raised intracranial pressure and herniation, and hydrocephalus; malformations and developmental diseases, such as neural tube defects, forebrain anomalies, posterior fossa anomalies, and syringomyelia and hydromyelia; perinatal brain injury; cerebrovascular diseases, such as those related to hypoxia, ischemia, and infarction, including hypotension, hypoperfusion, and low-flow states--global cerebral ischemia and focal cerebral ischemia--infarction from obstruction of local blood supply, intracranial hemorrhage, including intracerebral (intraparenchymal) hemorrhage, subarachnoid hemorrhage and ruptured berry aneurysms, and vascular malformations, hypertensive cerebrovascular disease, including lacunar infarcts, slit hemorrhages, and hypertensive encephalopathy; infections, such as acute meningitis, including acute pyogenic (bacterial) meningitis and acute aseptic (viral) meningitis, acute focal suppurative infections, including brain abscess, subdural empyema, and extradural abscess, chronic bacterial meningoencephalitis, including tuberculosis and mycobacterioses, neurosyphilis, and neuroborreliosis (Lyme disease), viral meningoencephalitis, including arthropod-borne (Arbo) viral encephalitis, *Herpes simplex* virus Type 1, *Herpes simplex* virus Type 2, *Varicalla-zoster* virus (*Herpes zoster*), cytomegalovirus, poliomyelitis, rabies, and human immunodeficiency virus 1, including HIV-1 meningoencephalitis (subacute encephalitis), vacuolar myelopathy, AIDS-associated myopathy, peripheral neuropathy, and AIDS in children, progressive multifocal leukoencephalopathy, subacute sclerosing panencephalitis, fungal meningoencephalitis, other infectious diseases of the nervous system; transmissible spongiform encephalopathies (prion diseases); demyelinating diseases, including multiple sclerosis, multiple sclerosis variants, acute disseminated encephalomyelitis and acute necrotizing hemorrhagic encephalomyelitis, and other diseases with demyelination; degenerative diseases, such as degenerative diseases affecting the cerebral cortex, including Alzheimer disease and Pick disease, degenerative diseases of basal ganglia and brain stem, including Parkinsonism, idiopathic Parkinson disease (paralysis agitans), progressive supranuclear palsy, corticobasal degenration, multiple system atrophy, including striatonigral degenration, Shy-Drager syndrome, and olivopontocerebellar atrophy, and Huntington disease; spinocerebellar degenerations, including spinocerebellar ataxias, including Friedreich ataxia, and ataxia-telanglectasia, degenerative diseases affecting motor neurons, including amyotrophic lateral sclerosis (motor neuron disease), bulbospinal atrophy (Kennedy syndrome), and spinal muscular atrophy; inborn errors of metabolism, such as leukodystrophies, including Krabbe disease, metachromatic leukodystrophy, adrenoleukodystrophy, Pelizaeus-Merzbacher disease, and Canavan disease, mitochondrial encephalomyopathies, including Leigh disease and other mitochondrial encephalomyopathies; toxic and acquired metabolic diseases, including vitamin deficiencies such as thiamine (vitamin B₁) deficiency and vitamin B₁₂ deficiency, neurologic sequelae of metabolic disturbances, including hypoglycemia, hyperglycemia, and hepatic encephatopathy, toxic disorders, including carbon monoxide, methanol, ethanol, and radiation, including combined methotrexate and radiation-induced injury; tumors, such as gliomas, including astrocytoma, including fibrillary (diffuse) astrocytoma and glioblastoma multiforme, pilocytic astrocytoma, pleomorphic xanthoastrocytoma, and brain stem glioma, oligodendroglioma, and ependymoma and related paraventricular mass lesions, neuronal tumors, poorly differentiated neoplasms, including medulloblastoma, other parenchymal tumors, including primary brain lymphoma, germ cell tumors, and pineal parenchymal tumors, meningiomas, metastatic tumors, paraneoplastic syndromes, peripheral nerve sheath tumors, including schwannoma, neurofibroma, and malignant peripheral nerve sheath tumor (malignant schwannoma), and neurocutaneous syndromes (phakomatoses), including neurofibromotosis, including Type 1 neurofibromatosis (NF1) and TYPE 2 neurofibromatosis (NF2), tuberous sclerosis, and Von Hippel-Lindau disease.

Disorders involving T-cells include, but are not limited to, cell-mediated hypersensitivity, such as delayed type hypersensitivity and T-cell-mediated cytotoxicity, and transplant rejection; autoimmune diseases, such as systemic lupus erythematosus, Sjögren syndrome, systemic sclerosis, inflammatory myopathies, mixed connective tissue disease, and polyarteritis nodosa and other vasculitides; immunologic deficiency syndromes, including but not limited to, primary immunodeficiencies, such as thymic hypoplasia, severe combined immunodeficiency diseases, and AIDS; leukopenia; reactive (inflammatory) proliferations of white cells, including but not limited to, leukocytosis, acute nonspecific lymphadenitis, and chronic nonspecific lymphadenitis; neoplastic proliferations of white cells, including but not limited to lymphoid neoplasms, such as precursor T-cell neoplasms, such as acute lymphoblastic leukemia/lymphoma, peripheral T-cell and natural killer cell neoplasms that include peripheral T-cell lymphoma, unspecified, adult T-cell leukemia/lymphoma, mycosis fungoides and Sézary syndrome, and Hodgkin disease.

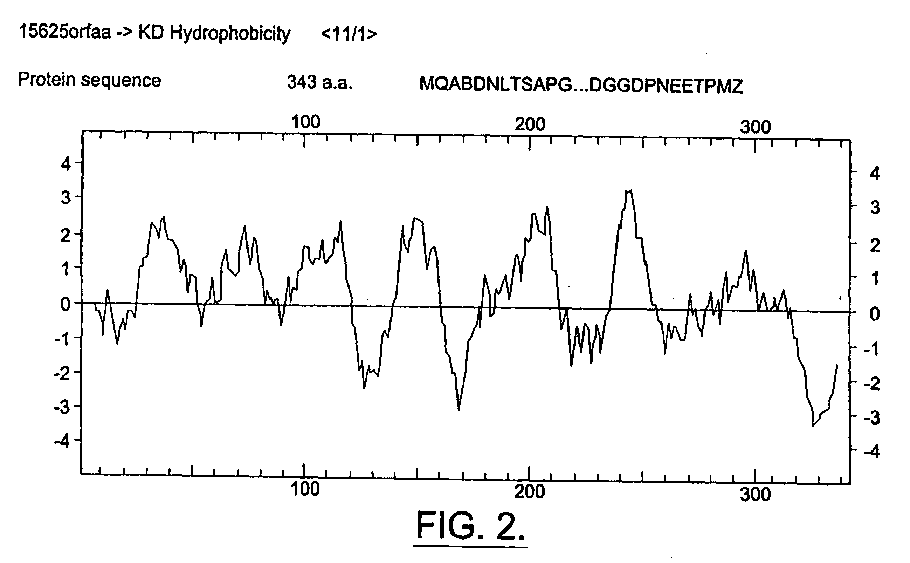

In normal bone marrow, the myelocytic series (polymorphoneuclear cells) make up approximately 60% of the cellular elements, and the erythrocytic series, 20-30%. Lymphocytes, monocytes, reticular cells, plasma cells and megakaryocytes together constitute 10-20%. Lymphocytes make up 5-15% of normal adult marrow. In the bone marrow, cell types are add mixed so that precursors of red blood cells (erythroblasts), macrophages (monoblasts), platelets (megakaryocytes), polymorphoneuclear leucocytes (myeloblasts), and lymphocytes (lymphoblasts) can be visible in one microscopic field. In addition, stem cells exist for the different cell lineages, as well as a precursor stem cell for the committed progenitor cells of the different lineages. The various types of cells and stages of each would be known to the person of ordinary skill in the art and are found, for example, on page 42 (Figure 2-8) of *Immunology, Imunopathology and Immunity,* Fifth Edition, Sell *et al.* Simon and Schuster (1996) disclosing teaching of cell types found in the bone marrow. According, the invention is directed to disorders arising from these cells. These disorders include but are not limited to the following: diseases involving hematopoeitic stem cells; committed lymphoid progenitor cells; lymphoid cells including B and T-cells; committed myeloid progenitors, including monocytes, granulocytes, and megakaryocytes; and committed erythroid progenitors. These include but are not limited to the leukemias, including B-lymphoid leukemias, T-lymphoid leukemias, undifferentiated leukemias; erythroleukemia, megakaryoblastic leukemia, monocytic leukemias are encompassed with and without differentiation; chronic and acute lymphoblastic leukemia, chronic and acute lymphocytic leukemia, chronic and acute myelogenous leukemia, lymphoma, myelo dysplastic syndrome, chronic and acute myeloid leukemia, myelomonocytic leukemia; chronic and acute myeloblastic leukemia, chronic and acute myelogenous leukemia, chronic and acute promyelocytic leukemia, chronic and acute myelocytic leukemia, hematologic malignancies of monocyte-macrophage lineage, such as juvenile chronic myelogenous leukemia; secondary AML, antecedent hematological disorder; refractory anemia; aplastic anemia; reactive cutaneous angioendotheliomatosis; fibrosing disorders involving altered expression in dendritic cells, disorders including systemic sclerosis, E-M syndrome, epidemic toxic oil syndrome, eosinophilic fasciitis localized forms of scleroderma, keloid, and fibrosing colonopathy; angiomatoid malignant fibrous histiocytoma; carcinoma, including primary head and neck squamous cell carcinoma; sarcoma, including kaposi's sarcoma; fibroadanoma and phyllodes tumors, including mammary fibroadenoma; stromal tumors; phyllodes tumors, including histiocytoma; erythroblastosis; neurofibromatosis; diseases of the vascular endothelium; demyelinating, particularly in old lesions; gliosis, vasogenic edema, vascular disease, Alzheimer's and Parkinson's disease; T-cell lymphomas; B-cell lymphomas.

Disorders involving the heart, include but are not limited to, heart failure, including but not limited to, cardiac hypertrophy, left-sided heart failure, and right-sided heart failure; ischemic heart disease, including but not limited to angina pectoris, myocardial infarction, chronic ischemic heart disease, and sudden cardiac death; hypertensive heart disease, including but not limited to, systemic (left-sided) hypertensive heart disease and pulmonary (right-sided) hypertensive heart disease; valvular heart disease, including but not limited to, valvular degeneration caused by calcification, such as calcific aortic stenosis, calcification of a congenitally bicuspid aortic valve, and mitral annular calcification, and myxomatous degeneration of the mitral valve (mitral valve prolapse), rheumatic fever and rheumatic heart disease, infective endocarditis, and noninfected vegetations, such as nonbacterial thrombotic endocarditis and endocarditis of systemic lupus erythematosus (Libman-Sacks disease), carcinoid heart disease, and complications of artificial valves; myocardial disease, including but not limited to dilated cardiomyopathy, hypertrophic cardiomyopathy, restrictive cardiomyopathy, and myocarditis; pericardial disease, including but not limited to, pericardial effusion and hemopericardium and pericarditis, including acute pericarditis and healed pericarditis, and rheumatoid heart disease; neoplastic heart disease, including but not limited to, primary cardiac tumors, such as myxoma, lipoma, papillary fibroelastoma, rhabdomyoma, and sarcoma, and cardiac effects of noncardiac neoplasms; congenital heart disease, including but not limited to, left-to-right shunts--late cyanosis, such as atrial septal defect, ventricular septal defect, patent ductus arteriosus, and atrioventricular septal defect, right-to-left shunts--early cyanosis, such as tetralogy of fallot, transposition of great arteries, truncus arteriosus, tricuspid atresia, and total anomalous pulmonary venous connection, obstructive congenital anomalies, such as coarctation of aorta, pulmonary stenosis and atresia, and aortic stenosis and atresia, and disorders involving cardiac transplantation.

Disorders involving red cells include, but are not limited to, anemias, such as hemolytic anemias, including hereditary spherocytosis, hemolytic disease due to erythrocyte enzyme defects: glucose-6-phosphate dehydrogenase deficiency, sickle cell disease, thalassemia syndromes, paroxysmal nocturnal hemoglobinuria, immunohemolytic anemia, and hemolytic anemia resulting from trauma to red cells; and anemias of diminished erythropoiesis, including megaloblastic anemias, such as anemias of vitamin B 12 deficiency: pernicious anemia, and anemia of folate deficiency, iron deficiency anemia, anemia of chronic disease, aplastic anemia, pure red cell aplasia, and other forms of marrow failure.

Disorders involving the thymus include developmental disorders, such as DiGeorge syndrome with thymic hypoplasia or aplasia; thymic cysts; thymic hypoplasia, which involves the appearance of lymphoid follicles within the thymus, creating thymic follicular hyperplasia; and thymomas, including germ cell tumors, lynphomas, Hodgkin disease, and carcinoids. Thymomas can include benign or encapsulated thymoma, and malignant thymoma Type I (invasive thymoma) or Type II, designated thymic carcinoma.

Disorders involving B-cells include, but are not limited to precursor B-cell neoplasms, such as lymphoblastic leukemia/lymphoma. Peripheral B-cell neoplasms include, but are not limited to, chronic lymphocytic leukemia/small lymphocytic lymphoma, follicular lymphoma, diffuse large B-cell lymphoma, Burkitt lymphoma, plasma cell neoplasms, multiple myeloma, and related entities, lymphoplasmacytic lymphoma (Waldenstro̅m macroglobulinemia), mantle cell lymphoma, marginal zone lymphoma (MALToma), and hairy cell leukemia.

Disorders involving the skeletal muscle include tumors such as rhabdomyosarcoma

Disorders involving the pancreas include those of the exocrine pancreas such as congenital anomalies, including but not limited to, ectopic pancreas; pancreatitis, including but not limited to, acute pancreatitis; cysts, including but not limited to, pseudocysts; tumors, including but not limited to, cystic tumors and carcinoma of the pancreas; and disorders of the endocrine pancreas such as, diabetes mellitus; islet cell tumors, including but not limited to, insulinomas, gastrinomas, and other rare islet cell tumors.

Disorders related to reduced platelet number, thrombocytopenia, include idiopathic thrombocytopenic purpura, including acute idiopathic thrombocytopenic purpura, drug-induced thrombocytopenia, HIV-associated thrombocytopenia, and thrombotic microangiopathies: thrombotic thrombocytopenic purpura and hemolytic-uremic syndrome.

Disorders involving precursor T-cell neoplasms include precursor T lymphoblastic leukemia/lymphoma. Disorders involving peripheral T-cell and natural killer cell neoplasms include T-cell chronic lymphocytic leukemia, large granular lymphocytic leukemia, mycosis fungoides and Sézary syndrome, peripheral T-cell lymphoma, unspecified, angioimmunoblastic T-cell lymphoma, angiocentric lymphoma (NK/T-cell lymphoma^{4a}), intestinal T-cell lymphoma, adult T-cell leukemia/lymphoma, and anaplastic large cell lymphoma.

The gene is expressed at significant levels in all blood cell progenitors analyzed by the inventors. It is highly expressed in bone marrow (CD34⁺), G-CSF-mobilized peripheral blood (containing circulating progenitors derived from bone marrow) and cord blood progenitors. Accordingly, expression of the gene is relevant for treating disorders associated with the formation of differentiated and/or mature blood cells. In this regard, disorders that are particularly relevant include anemia, neutropenia, and thrombocytopenia.

The receptor polypeptides also are useful to provide a target for diagnosing a disease or predisposition to disease mediated by the receptor protein, as discussed above regarding treatment, especially in brain (especially glial cells) and bone marrow CD34⁺ cells, including but not limited to, megakaryocytes, and also in heart, placenta, pancreas, resting B lymphocytes, skeletal muscle, lymph node, spleen, thymus, liver, tonsils, colon, granulocytes and erythroblasts. Accordingly, methods are provided for detecting the presence, or levels of, the receptor protein in a cell, tissue, or organism. The method involves contacting a biological sample with a compound capable of interacting with the receptor protein such that the interaction can be detected.

One agent for detecting receptor protein is an antibody capable of selectively binding to receptor protein. A biological sample includes tissues, cells and biological fluids isolated from a subject, as well as tissues, cells and fluids present within a subject.

The receptor protein also provides a target for diagnosing active disease, or predisposition to disease, in a patient having a variant receptor protein. Thus, receptor protein can be isolated from a biological sample, assayed for the presence of a genetic mutation that results in aberrant receptor protein. This includes amino acid substitution, deletion, insertion, rearrangement, (as the result of aberrant splicing events), and inappropriate post-translational modification. Analytic methods include altered electrophoretic mobility, altered tryptic peptide digest, altered receptor activity in cell-based or cell-free assay, alteration in ligand or antibody-binding pattern, altered isoelectric point, direct amino acid sequencing, and any other of the known assay techniques useful for detecting mutations in a protein.

*In vitro* techniques for detection of receptor protein include enzyme linked immunosorbent assays (ELISAs), Western blots, immunoprecipitations and immunofluorescence. Alternatively, the protein can be detected *in vivo* in a subject by introducing into the subject a labeled anti-receptor antibody. For example, the antibody can be labeled with a radioactive marker whose presence and location in a subject can be detected by standard imaging techniques. Particularly useful are methods which detect the allelic variant of a receptor protein expressed in a subject and methods which detect fragments of a receptor protein in a sample.

The receptor polypeptides are also useful in pharmacogenomic analysis. Pharmacogenomics deal with clinically significant hereditary variations in the response to drugs due to altered drug disposition and abnormal action in affected persons. See, e.g., Eichelbaum, M. (*Clin. Exp. Pharmacol. Physiol.* 23(10-11) :983-985 (1996)), and Linder, M.W. (*Clin. Chem.* 43(2):254-266 (1997)). The clinical outcomes of these variations result in severe toxicity of therapeutic drugs in certain individuals or therapeutic failure of drugs in certain individuals as a result of individual variation in metabolism. Thus, the genotype of the individual can determine the way a therapeutic compound acts on the body or the way the body metabolizes the compound. Further, the activity of drug metabolizing enzymes effects both the intensity and duration of drug action. Thus, the pharmacogenomics of the individual permit the selection of effective compounds and effective dosages of such compounds for prophylactic or therapeutic treatment based on the individual's genotype. The discovery of genetic polymorphisms in some drug metabolizing enzymes has explained why some patients do not obtain the expected drug effects, show an exaggerated drug effect, or experience serious toxicity from standard drug dosages. Polymorphisms can be expressed in the phenotype of the extensive metabolizer and the phenotype of the poor metabolizer. Accordingly, genetic polymorphism may lead to allelic protein variants of the receptor protein in which one or more of the receptor functions in one population is different from those in another population. The polypeptides thus allow a target to ascertain a genetic predisposition that can affect treatment modality. Thus, in a ligand-based treatment, polymorphism may give rise to amino terminal extracellular domains and/or other ligand-binding regions that are more or less active in ligand binding, and receptor activation. Accordingly, ligand dosage would necessarily be modified to maximize the therapeutic effect within a given population containing a polymorphism. As an alternative to genotyping, specific polymorphic polypeptides could be identified.

The receptor polypeptides are also useful for monitoring therapeutic effects during clinical trials and other treatment. Thus, the therapeutic effectiveness of an agent that is designed to increase or decrease gene expression, protein levels or receptor activity can be monitored over the course of treatment using the receptor polypeptides as an end-point target.

The receptor polypeptides are also useful for treating a receptor-associated disorder. Accordingly, methods for treatment include the use of soluble receptor or fragments of the receptor protein that compete for ligand binding. These receptors or fragments can have a higher affinity for the ligand so as to provide effective competition.

The disclosure above generally applies also to the sequence shown in SEQ ID NO 3.

### Antibodies

The invention also provides antibodies that selectively bind to the 15625 receptor protein and its variants and fragments. An antibody is considered to selectively bind, even if it also binds to other proteins that are not substantially homologous with the receptor protein. These other proteins share homology with a fragment or domain of the receptor protein. This conservation in specific regions gives rise to antibodies that bind to both proteins by virtue of the homologous sequence. In this case, it would be understood that antibody binding to the receptor protein is still selective.

To generate antibodies, an isolated receptor polypeptide is used as an immunogen to generate antibodies using standard techniques for polyclonal and monoclonal antibody preparation. Either the full-length protein or antigenic peptide fragment can be used. Regions having a high antigenicity index are shown in Figure 1.

Antibodies are preferably prepared from these regions or from discrete fragments in these regions. However, antibodies can be prepared from any region of the peptide as described herein. A preferred fragment produces an antibody that diminishes or completely prevents ligand-binding. Antibodies can be developed against the entire receptor or portions of the receptor, for example, the intracellular carboxy terminal domain, the amino terminal extracellular domain, the entire transmembrane domain or specific segments, any of the intra or extracellular loops, or any portions of the above. Antibodies may also be developed against specific functional sites, such as the site of ligand-binding, the site of G protein coupling, or sites that are glycosylated, phosphorylated, or myristoylated.

An antigenic fragment will typically comprise at least 10 contiguous amino acid residues. The antigenic peptide can comprise, however, at least 12, at least 14 amino acid residues, at least 15 amino acid residues, at least 20 amino acid residues, or at least 30 amino acid residues. In one embodiment, fragments correspond to regions that are located on the surface of the protein, e.g., hydrophilic regions. These fragments are not to be construed, however, as encompassing any fragments which may be disclosed prior to the invention.

Antibodies can be.polyclonal or monoclonal. An intact antibody, or a fragment thereof (e.g. Fab or F(ab')₂) can be used.

Detection can be facilitated by coupling (i.e., physically linking) the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

An appropriate immunogenic preparation can be derived from native, recombinantly expressed, protein or chemically synthesized peptides.

### Antibody Uses

The antibodies can be used to isolate a receptor protein by standard techniques, such as affinity chromatography or immunoprecipitation. The antibodies can facilitate the purification of the natural receptor protein from cells and recombinantly produced receptor protein expressed in host cells.

The antibodies are useful to detect the presence of receptor protein in cells or tissues to determine the pattern of expression of the receptor among various tissues in an organism and over the course of normal development.

The antibodies can be used to detect receptor protein *in situ, in vitro,* or in a cell lysate or supernatant in order to evaluate the abundance and pattern of expression.

The antibodies can be used to assess abnormal tissue distribution or abnormal expression during development.

Antibody detection of circulating fragments of the full length receptor protein can be used to identify receptor turnover.

Further, the antibodies can be used to assess receptor expression in disease states such as in active stages of the disease or in an individual with a predisposition toward disease related to receptor function. When a disorder is caused by an inappropriate tissue distribution, developmental expression, or level of expression of the receptor protein, the antibody can be prepared against the normal receptor protein. If a disorder is characterized by a specific mutation in the receptor protein, antibodies specific for this mutant protein can be used to assay for the presence of the specific mutant receptor protein. However, intracellularly-made antibodies ("intrabodies") are also encompassed, which would recognize intracellular receptor peptide regions.

The antibodies can also be used to assess normal and aberrant subcellular localization of cells in the various tissues in an organism. Antibodies can be developed against the whole receptor or portions of the receptor, for example, portions of the amino terminal extracellular domain or extracellular loops.

The diagnostic uses can be applied, not only in genetic testing, but also in monitoring a treatment modality. Accordingly, where treatment is ultimately aimed at correcting receptor expression level or the presence of aberrant receptors and aberrant tissue distribution or developmental expression, antibodies directed against the receptor or relevant fragments can be used to monitor therapeutic efficacy.

Additionally, antibodies are useful in pharmacogenomic analysis. Thus, antibodies prepared against polymorphic receptor proteins can be used to identify individuals that require modified treatment modalities.

The antibodies are also useful as diagnostic tools as an immunological marker for aberrant receptor protein analyzed by electrophoretic mobility, isoelectric point, tryptic peptide digest, and other physical assays known to those in the art.

The antibodies are also useful for tissue typing. Thus, where a specific receptor protein has been correlated with expression in a specific tissue, antibodies that are specific for this receptor protein can be used to identify a tissue type.

The antibodies are also useful in forensic identification. Accordingly, where an individual has been correlated with a specific genetic polymorphism resulting in a specific polymorphic protein, an antibody specific for the polymorphic protein can be used as an aid in identification.

The antibodies are also useful for inhibiting receptor function, for example, blocking ligand binding.

These uses can also be applied in a therapeutic context in which treatment involves inhibiting receptor function. An antibody can be used, for example, to block ligand binding. Antibodies can be prepared against specific fragments containing sites required for function or against intact receptor associated with a cell.

The invention also encompasses kits for using antibodies to detect the presence of a receptor protein in a biological sample. The kit can comprise antibodies such as a labeled or labelable antibody and a compound or agent for detecting receptor protein in a biological sample; means for determining the amount of receptor protein in the sample; and means for comparing the amount of receptor protein in the sample with a standard. The compound or agent can be packaged in a suitable container. The kit can further comprise instructions for using the kit to detect receptor protein.

The above disclosure generally applies also to the sequence shown in SEQ ID NO 3.

### Polynucleotides

The specifically disclosed cDNA comprises the coding region and 5' and 3' untranslated sequences (SEQ ID NO 2).

The human 15625 receptor cDNA is approximately 2286 nucleotides in length and encodes a full length protein that is approximately 342 amino acid residues in length. The nucleic acid is expressed as disclosed herein, such as in brain, especially glial cells, CD34⁺ cells, and 293 and Jurkat cell lines. Structural analysis of the amino acid sequence of SEQ ID NO 1 is provided in Figure 2, a hydropathy plot. The figure shows the putative structure of the seven transmembrane segments, the amino terminal extracellular domain and the carboxy terminal intracellular domain.

As used herein, the term "transmembrane segment" refers to a structural amino acid motif which includes a hydrophobic helix that spans the plasma membrane. The entire transmembrane domain spans from about amino acid 26 to about amino acid 302. Seven segments span the membrane and there are three intracellular and three extracellular loops in this domain.

The invention provides isolated polynucleotides encoding a 15625 receptor protein. The term "15625 polynucleotide" or "15625 nucleic acid" refers to the sequence shown in SEQ ID NO 2. The term "receptor polynucleotide" or "receptor nucleic acid" further includes variants and fragments of the 15625 polynucleotide, such as that shown in SEQ ID NO 4.

An "isolated" receptor nucleic acid is one that is separated from other nucleic acid present in the natural source of the receptor nucleic acid. Preferably, an "isolated" nucleic acid is free of sequences which naturally flank the nucleic acid (i.e., sequences located at the 5' and 3' ends of the nucleic acid) in the genomic DNA of the organism from which the nucleic acid is derived. However, there can be some flanking nucleotide sequences, for example up to about 5KB. The important point is that the nucleic acid is isolated from flanking sequences such that it can be subjected to the specific manipulations described herein such as recombinant expression, preparation of probes and primers, and other uses specific to the receptor nucleic acid sequences.

Moreover, an "isolated" nucleic acid molecule, such as a cDNA molecule, can be substantially free of other cellular material, or culture medium when produced by recombinant techniques, or chemical precursors or other chemicals when chemically synthesized. However, the nucleic acid molecule can be fused to other coding or regulatory sequences and still be considered isolated.

For example, recombinant DNA molecules contained in a vector are considered isolated. Further examples of isolated DNA molecules include recombinant DNA molecules maintained in heterologous host cells or purified (partially or substantially) DNA molecules in solution. Isolated RNA molecules include *in vivo* or *in vitro* RNA transcripts of the isolated DNA molecules of the present invention. Isolated nucleic acid molecules according to the present invention further include such molecules produced synthetically.

The receptor polynucleotides can encode the mature protein plus additional amino or carboxyl-terminal amino acids, or amino acids interior to the mature polypeptide (when the mature form has more than one polypeptide chain, for instance). Such sequences may play a role in processing of a protein from precursor to a mature form, facilitate protein trafficking, prolong or shorten protein half-life or facilitate manipulation of a protein for assay or production, among other things. As generally is the case *in situ,* the additional amino acids may be processed away from the mature protein by cellular enzymes.

The receptor polynucleotides include, but are not limited to, the sequence encoding the mature polypeptide alone, the sequence encoding the mature polypeptide and additional coding sequences, such as a leader or secretory sequence (e.g., a pre-pro or pro-protein sequence), the sequence encoding the mature polypeptide, with or without the additional coding sequences, plus additional non-coding sequences, for example introns and non-coding 5' and 3' sequences such as transcribed but non-translated sequences that play a role in transcription, mRNA processing (including splicing and polyadenylation signals), ribosome binding and stability of mRNA. In addition, the polynucleotide may be fused to a marker sequence encoding, for example, a peptide that facilitates purification.

Receptor polynucleotides can be in the form of RNA, such as mRNA, or in the form DNA, including cDNA and genomic DNA obtained by cloning or produced by chemical synthetic techniques or by a combination thereof The nucleic acid, especially DNA, can be double-stranded or single-stranded. Single-stranded nucleic acid can be the coding strand (sense strand) or the non-coding strand (anti-sense strand).

One receptor nucleic acid comprises the nucleotide sequence shown in SEQ ID NO 2, corresponding to human brain cDNA.

In one embodiment, the receptor nucleic acid comprises only the coding region.

The invention further provides variant receptor polynucleotides, and fragments thereof, that differ from the nucleotide sequence shown in SEQ ID NO 2 due to degeneracy of the genetic code and thus encode the same protein as that encoded by the nucleotide sequence shown in SEQ ID NO 2.

The invention also provides receptor nucleic acid molecules encoding the variant polypeptides described herein. Such polynucleotides may be naturally occurring, such as allelic variants (same locus) (maps to chromosome 3 near AFM164YG9), homologs (different locus), and orthologs (different organism), such as the sequence shown in SEQ ID NO 4, or may be constructed by recombinant DNA methods or by chemical synthesis. Such non-naturally occurring variants may be made by mutagenesis techniques, including those applied to polynucleotides, cells, or organisms. Accordingly, as discussed above, the variants can contain nucleotide substitutions, deletions, inversions and insertions.

Variation can occur in either or both the coding and non-coding regions. The variations can produce both conservative and non-conservative amino acid substitutions.

Orthologs, homologs, and allelic variants can be identified using methods well known in the art. These variants comprise a nucleotide sequence encoding a receptor that is 50%, at least about 55%, typically at least about 70-75%, more typically at least about 80-85%, and most typically at least about 90-95% or more homologous to the nucleotide sequence shown in SEQ ID NO 2 or a fragment of this sequence. Such nucleic acid molecules can readily be identified as being able to hybridize under stringent conditions, to the nucleotide sequence shown in SEQ ID NO 2 or a fragment of the sequence. It is understood that stringent hybridization does not indicate substantial homology where it is due to general homology, such as poly A sequences, or sequences common to all or most proteins, all GPCRs, or all family I GPCRs. Moreover, it is understood that variants do not include any of the nucleic acid sequences that may have been disclosed prior to the invention.

As used herein, the term "hybridizes under stringent conditions" is intended to describe conditions for hybridization and washing under which nucleotide sequences encoding a receptor at least 50%, 55% homologous to each other typically remain hybridized to each other. The conditions can be such that sequences at least about 65%, at least about 70%, or at least about 75% or more homologous to each other typically remain hybridized to each other. Such stringent conditions are known to those skilled in the art and can be found in *Current Protocols in Molecular Biology,* John Wiley & Sons, N.Y. (1989), 6.3.1-6.3.6. One example of stringent hybridization conditions are hybridization in 6X sodium chloride/sodium citrate (SSC) at about 45°C, followed by one or more washes in 0.2 X SSC, 0.1% SDS at 50-65°C. In one embodiment, an isolated receptor nucleic acid molecule that hybridizes under stringent conditions to the sequence of SEQ ID NO 2 corresponds to a naturally-occurring nucleic acid molecule. As used herein, a "naturally-occurring" nucleic acid molecule refers to an RNA or DNA molecule having a nucleotide sequence that occurs in nature (e.g., encodes a natural protein).

Furthermore, the invention provides polynucleotides that comprise a fragment of the full length receptor polynucleotides. The fragment can be single or double stranded and can comprise DNA or RNA. The fragment can be derived from either the coding or the non-coding sequence.

A fragment can comprise a contiguous nucleotide sequence greater than 12 nucleotides from nucleotide 1 to about nucleotide 500, greater than 24 nucleotides from about nucleotide 476 to about nucleotide 1096, and greater than 12 nucleotides from about nucleotide 1147 to nucleotide 1715.

Isolated receptor nucleic acid fragments hybridize under stringent conditions to the nucleic acid molecule comprising the nucleotide sequence of SEQ ID NO 2. In other embodiments, the nucleic acid is at least 30, 40, 50, 100, 250 or 500 nucleotides in length.

In another embodiment an isolated receptor nucleic acid encodes the entire coding region from amino acid 1 to amino acid 342. In another embodiment the isolated receptor nucleic acid encodes a sequence corresponding to the mature protein from about amino acid 6 to amino acid 342. Other fragments include nucleotide sequences encoding the amino acid fragments described herein. Further fragments can include subfragments of the specific domains or sites described herein. Nucleic acid fragments, according to the present invention, are not to be construed as encompassing those fragments that may have been disclosed prior to the invention.

Receptor nucleic acid fragments further include sequences corresponding to the domains described herein, subregions also described, and specific functional sites. Receptor nucleic acid fragments also include combinations of the domains, segments, loops, and other functional sites described above. Thus, for example, a receptor nucleic acid could include sequences corresponding to the amino terminal extracellular domain and one transmembrane fragment. A person of ordinary skill in the art would be aware of the many permutations that are possible.

However, it is understood that a receptor fragment includes any nucleic acid sequence that does not include the entire gene.

Receptor nucleic acid fragments include nucleic acid molecules encoding a polypeptide comprising the amino terminal extracellular domain including amino acid residues from 1 to about 25, a polypeptide comprising the region spanning the transmembrane domain (amino acid residues from about 26 to about 302), a polypeptide comprising the carboxy terminal intracellular domain (amino acid residues from about 303 to about 342), and a polypeptide encoding the G-protein receptor signature (121-123 or surrounding amino acid residues from about 110 to about 133), nucleic acid molecules encoding any of the seven transmembrane segments, extracellular or intracellular loops, and sites for glycosylation, cAMP and cGMP-dependent protein kinase phosphorylation, protein kinase C phosphorylation, and N-myristoylation. Where the location of the domains have been predicted by computer analysis, one of ordinary skill would appreciate that the amino acid residues constituting these domains can vary depending on the criteria used to define the domains.

The invention also provides receptor nucleic acid fragments that encode epitope bearing regions of the receptor proteins described herein.

The isolated receptor polynucleotide sequences, and especially fragments, are useful as DNA probes and primers.

For example, the coding region of a receptor gene can be isolated using the known nucleotide sequence to synthesize an oligonucleotide probe. A labeled probe can then be used to screen a cDNA library, genomic DNA library, or mRNA to isolate nucleic acid corresponding to the coding region. Further, primers can be used in PCR reactions to clone specific regions of receptor genes.

A probe/primer typically comprises substantially purified oligonucleotide. The oligonucleotide typically comprises a region of nucleotide sequence that hybridizes under stringent conditions to at least about 12, typically about 25, more typically about 40, 50 or 75 consecutive nucleotides of SEQ ID NO 2 sense or anti-sense strand or other receptor polynucleotides. A probe further comprises a label, e.g., radioisotope, fluorescent compound, enzyme, or enzyme co-factor.

The above disclosure generally also applies to the sequence shown in SEQ ID NO 4.

### Polynucleotide Uses

The receptor polynucleotides are useful for probes, primers, and in biological assays. Where the polynucleotides are used to assess GPCR properties or properties or functions, such as in the assays described herein, all or less than all of the entire cDNA can be useful. In this case, even fragments that may have been known prior to the invention are encompassed. Thus, for example, assays specifically directed to GPCR functions, such as assessing agonist or antagonist activity, encompass the use of known fragments. Further, diagnostic methods for assessing receptor function can also be practiced with any fragment, including those fragments that may have been known prior to the invention. Similarly, in methods involving treatment of receptor dysfunction, all fragments are encompassed including those which may have been known in the art.

The receptor polynucleotides are useful as a hybridization probe for cDNA and genomic DNA to isolate a full-length cDNA and genomic clones encoding the polypeptide described in SEQ ID NO 1 and to isolate cDNA and genomic clones that correspond to variants producing the same polypeptide shown in SEQ ID NO 1 or the other variants described herein. Variants can be isolated from the same tissue and organism from which the polypeptide shown in SEQ ID NO 1 was isolated, different tissues from the same organism, or from different organisms. This method is useful for isolating genes and cDNA that are developmentally-controlled and therefore may be expressed in the same tissue or different tissues at different points in the development of an organism.

The probe can correspond to any sequence along the entire length of the gene encoding the receptor. Accordingly, it could be derived from 5' noncoding regions, the coding region, and 3' noncoding regions. However, as discussed, fragments are not to be construed as those which may encompass fragments disclosed prior to the present invention.

The nucleic acid probe can be, for example, the full-length cDNA of SEQ ID NO 1, or a fragment thereof, such as an oligonucleotide of at least 12,15, 30, 50, 100, 250 or 500 nucleotides in length and sufficient to specifically hybridize under stringent conditions to mRNA or DNA.

Fragments of the polynucleotides described herein are also useful to synthesize larger fragments or full-length polynucleotides described herein. For example, a fragment can be hybridized to any portion of an mRNA and a larger or full-length cDNA can be produced.

The fragments are also useful to synthesize antisense molecules of desired length and sequence.

The receptor polynucleotides are also useful as primers for PCR to amplify any given region of a receptor polynucleotide.

The receptor polynucleotides are also useful for constructing recombinant vectors. Such vectors include expression vectors that express a portion of, or all of, the receptor polypeptides. Vectors also include insertion vectors, used to integrate into another polynucleotide sequence, such as into the cellular genome, to alter *in situ* expression of receptor genes and gene products. For example, an endogenous receptor coding sequence can be replaced via homologous recombination with all or part of the coding region containing one or more specifically introduced mutations.

The receptor polynucleotides are also useful for expressing antigenic portions of the receptor proteins.

The receptor polynucleotides are also useful as probes for determining the chromosomal positions of the receptor polynucleotides by means of *in situ* hybridization methods.

The receptor polynucleotide probes are also useful to determine patterns of the presence of the gene encoding the receptors and their variants with respect to tissue distribution, for example, whether gene duplication has occurred and whether the duplication occurs in all or only a subset of tissues. The genes can be naturally occurring or can have been introduced into a cell, tissue, or organism exogenously.

The receptor polynucleotides are also useful for designing ribozymes corresponding to all, or a part, of the mRNA produced from genes encoding the polynucleotides described herein.

The receptor polynucleotides are also useful for constructing host cells expressing a part, or all, of the receptor polynucleotides and polypeptides.

The receptor polynucleotides are also useful for constructing transgenic animals expressing all, or a part, of the receptor polynucleotides and polypeptides.

The receptor polynucleotides are also useful for making vectors that express part, or all, of the receptor polypeptides.

The receptor polynucleotides are also useful as hybridization probes for determining the level of receptor nucleic acid expression. Accordingly, the probes can be used to detect the presence of, or to determine levels of, receptor nucleic acid in cells, tissues, and in organisms. The nucleic acid whose level is determined can be DNA or RNA. Accordingly, probes corresponding to the polypeptides described herein can be used to assess gene copy number in a given cell, tissue, or organism. This is particularly relevant in cases in which there has been an amplification of the receptor genes.

Alternatively, the probe can be used in an *in situ* hybridization context to assess the position of extra copies of the receptor genes, as on extrachromosomal elements or as integrated into chromosomes in which the receptor gene is not normally found, for example as a homogeneously staining region.

These uses are relevant for diagnosis of disorders involving an increase or decrease in receptor expression relative to normal results.

*In vitro* techniques for detection of mRNA include Northern hybridizations and *in situ* hybridizations. *In vitro* techniques for detecting DNA includes Southern hybridizations and *in situ* hybridization.

Probes can be used as a part of a diagnostic test kit for identifying cells or tissues that express a receptor protein, such as by measuring a level of a receptor-encoding nucleic acid in a sample of cells from a subject e.g., mRNA or genomic DNA, or determining if a receptor gene has been mutated.

Nucleic acid expression assays are useful for drug screening to identify compounds that modulate receptor nucleic acid expression.

The invention thus provides a method for identifying a compound that can be used to treat a disorder associated with nucleic acid expression of the receptor gene. The method typically includes assaying the ability of the compound to modulate the expression of the receptor nucleic acid and thus identifying a compound that can be used to treat a disorder characterized by undesired receptor nucleic acid expression.

The assays can be performed in cell-based and cell-free systems. Cell-based assays include cells naturally expressing the receptor nucleic acid or recombinant cells genetically engineered to express specific nucleic acid sequences.

Alternatively, candidate compounds can be assayed *in vivo* in patients or in transgenic animals.

The assay for receptor nucleic acid expression can involve direct assay of nucleic acid levels, such as mRNA levels, or on collateral compounds involved in the signal pathway (such as cAMP or phosphatidylinositol turnover). Further, the expression of genes that are up- or down-regulated in response to the receptor protein signal pathway can also be assayed. In this embodiment the regulatory regions of these genes can be operably linked to a reporter gene such as luciferase.

Thus, modulators of receptor gene expression can be identified in a method wherein a cell is contacted with a candidate compound and the expression of mRNA determined. The level of expression of receptor mRNA in the presence of the candidate compound is compared to the level of expression of receptor mRNA in the absence of the candidate compound. The candidate compound can then be identified as a modulator of nucleic acid expression based on this comparison and be used, for example to treat a disorder characterized by aberrant nucleic acid expression. When expression of mRNA is statistically significantly greater in the presence of the candidate compound than in its absence, the candidate compound is identified as a stimulator of nucleic acid expression. When nucleic acid expression is statistically significantly less in the presence of the candidate compound than in its absence, the candidate compound is identified as an inhibitor of nucleic acid expression.

Accordingly, the invention provides methods of treatment, with the nucleic acid as a target, using a compound identified through drug screening as a gene modulator to modulate receptor nucleic acid expression. Modulation includes both up-regulation (i.e. activation or agonization) or down-regulation (suppression or antagonization) or nucleic acid expression.

Alternatively, a modulator for receptor nucleic acid expression can be a small molecule or drug identified using the screening assays described herein as long as the drug or small molecule inhibits the receptor nucleic acid expression.

The receptor polynucleotides are also useful for monitoring the effectiveness of modulating compounds on the expression or activity of the receptor gene in clinical trials or in a treatment regimen. Thus, the gene expression pattern can serve as a barometer for the continuing effectiveness of treatment with the compound, particularly with compounds to which a patient can develop resistance. The gene expression pattern can also serve as a marker indicative of a physiological response of the affected cells to the compound. Accordingly, such monitoring would allow either increased administration of the compound or the administration of alternative compounds to which the patient has not become resistant. Similarly, if the level of nucleic acid expression falls below a desirable level, administration of the compound could be commensurately decreased.

The receptor polynucleotides are also useful in diagnostic assays for qualitative changes in receptor nucleic acid, and particularly in qualitative changes that lead to pathology. The polynucleotides can be used to detect mutations in receptor genes and gene expression products such as mRNA. The polynucleotides can be used as hybridization probes to detect naturally-occurring genetic mutations in the receptor gene and thereby to determine whether a subject with the mutation is at risk for a disorder caused by the mutation. Mutations include deletion, addition, or substitution of one or more nucleotides in the gene, chromosomal rearrangement, such as inversion or transposition, modification of genomic DNA, such as aberrant methylation patterns or changes in gene copy number, such as amplification. Detection of a mutated form of the receptor gene associated with a dysfunction provides a diagnostic tool for an active disease or susceptibility to disease when the disease results from overexpression, underexpression, or altered expression of a receptor protein.

Individuals carrying mutations in the receptor gene can be detected at the nucleic acid level by a variety of techniques. Genomic DNA can be analyzed directly or can be amplified by using PCR prior to analysis. RNA or cDNA can be used in the same way.

In certain embodiments, detection of the mutation involves the use of a probe/primer in a polymerase chain reaction (PCR) (see, e.g. U.S. Patent Nos. 4,683,195 and 4,683,202), such as anchor PCR or RACE PCR, or, alternatively, in a ligation chain reaction (LCR) (see, e.g., Landegran *et al., Science 241*:1077-1080 (1988); and Nakazawa *et al., PNAS 91*:360-364 (1994)), the latter of which can be particularly useful for detecting point mutations in the gene (see Abravaya *et al., Nucleic Acids Res 23*:675-682 (1995)). This method can include the steps of collecting a sample of cells from a patient, isolating nucleic acid (e.g., genomic, mRNA or both) from the cells of the sample, contacting the nucleic acid sample with one or more primers which specifically hybridize to a gene under conditions such that hybridization and amplification of the gene (if present) occurs, and detecting the presence or absence of an amplification product, or detecting the size of the amplification product and comparing the length to a control sample. Deletions and insertions can be detected by a change in size of the amplified product compared to the normal genotype. Point mutations can be identified by hybridizing amplified DNA to normal RNA or antisense DNA sequences.

Alternatively, mutations in a receptor gene can be directly identified, for example, by alterations in restriction enzyme digestion patterns determined by gel electrophoresis.

Further, sequence-specific ribozymes (U.S. Patent No. 5,498,531) can be used to score for the presence of specific mutations by development or loss of a ribozyme cleavage site.

Perfectly matched sequences can be distinguished from mismatched sequences by nuclease cleavage digestion assays or by differences in melting temperature.

Sequence changes at specific locations can also be assessed by nuclease protection assays such as RNase and S1 protection or the chemical cleavage method.

Furthermore, sequence differences between a mutant receptor gene and a wild-type gene can be determined by direct DNA sequencing. A variety of automated sequencing procedures can be utilized when performing the diagnostic assays ((1995) *Biotechniques 19*:448), including sequencing by mass spectrometry (see, e.g., PCT International Publication No. WO 94/16101; Cohen *et al., Adv. Chromatogr. 36*:127-162 (1996); and Griffin *et al., Appl. Biochem. Biolechnol. 38*:147-159 (1993)).

Other methods for detecting mutations in the gene include methods in which protection from cleavage agents is used to detect mismatched bases in RNA/RNA or RNA/DNA duplexes (Myers *et al., Science 230*:1242 (1985)); Cotton *et al., PNAS 85*:4397 (1988); Saleeba *et al., Meth. Enzymol. 217*:286-295 (1992)), electrophoretic mobility of mutant and wild type nucleic acid is compared (Orita *et al., PNAS 86*:2766 (1989); Cotton *et al., Mutat. Res. 285*:125-144 (1993); and Hayashi *et al., Genet. Anal. Tech. Appl. 9*:73-79 (1992)), and movement of mutant or wild-type fragments in polyacrylamide gels containing a gradient of denaturant is assayed using denaturing gradient gel electrophoresis (Myers *et al., Nature 313*:495 (1985)). Examples of other techniques for detecting point mutations include, selective oligonucleotide hybridization, selective amplification, and selective primer extension.

The receptor polynucleotides are also useful for testing an individual for a genotype that while not necessarily causing the disease, nevertheless affects the treatment modality. Thus, the polynucleotides can be used to study the relationship between an individual's genotype and the individual's response to a compound used for treatment (pharmacogenomic relationship). In the present case, for example, a mutation in the receptor gene that results in altered affinity for ligand could result in an excessive or decreased drug effect with standard concentrations of ligand that activates the receptor. Accordingly, the receptor polynucleotides described herein can be used to assess the mutation content of the receptor gene in an individual in order to select an appropriate compound or dosage regimen for treatment.

Thus polynucleotides displaying genetic variations that affect treatment provide a diagnostic target that can be used to tailor treatment in an individual. Accordingly, the production of recombinant cells and animals containing these polymorphisms allow effective clinical design of treatment compounds and dosage regimens.

The receptor polynucleotides are also useful for chromosome identification when the sequence is identified with an individual chromosome and to a particular location on the chromosome. First, the DNA sequence is matched to the chromosome by *in situ* or other chromosome-specific hybridization. Sequences can also be correlated to specific chromosomes by preparing PCR primers that can be used for PCR screening of somatic cell hybrids containing individual chromosomes from the desired species. Only hybrids containing the chromosome containing the gene homologous to the primer will yield an amplified fragment. Sublocalization can be achieved using chromosomal fragments. Other strategies include prescreening with labeled flow-sorted chromosomes and preselection by hybridization to chromosome-specific libraries. Further mapping strategies include fluorescence *in situ* hybridization which allows hybridization with probes shorter than those traditionally used. Reagents for chromosome mapping can be used individually to mark a single chromosome or a single site on the chromosome, or panels of reagents can be used for marking multiple sites and/or multiple chromosomes. Reagents corresponding to noncoding regions of the genes actually are preferred for mapping purposes. Coding sequences are more likely to be conserved within gene families, thus increasing the chance of cross hybridizations during chromosomal mapping.

The receptor polynucleotides can also be used to identify individuals from small biological samples. This can be done for example using restriction fragment-length polymorphism (RFLP) to identify an individual. Thus, the polynucleotides described herein are useful as DNA markers for RFLP (See U.S. Patent No. 5,272,057).

Furthermore, the receptor sequence can be used to provide an alternative technique which determines the actual DNA sequence of selected fragments in the genome of an individual. Thus, the receptor sequences described herein can be used to prepare two PCR primers from the 5' and 3' ends of the sequences. These primers can then be used to amplify DNA from an individual for subsequent sequencing.

Panels of corresponding DNA sequences from individuals prepared in this manner can provide unique individual identifications, as each individual will have a unique set of such DNA sequences. It is estimated that allelic variation in humans occurs with a frequency of about once per each 500 bases. Allelic variation occurs to some degree in the coding regions of these sequences, and to a greater degree in the noncoding regions. The receptor sequences can be used to obtain such identification sequences from individuals and from tissue. The sequences represent unique fragments of the human genome. Each of the sequences described herein can, to some degree, be used as a standard against which DNA from an individual can be compared for identification purposes.

If a panel of reagents from the sequences is used to generate a unique identification database for an individual, those same reagents can later be used to identify tissue from that individual. Using the unique identification database, positive identification of the individual, living or dead, can be made from extremely small tissue samples.

The receptor polynucleotides can also be used in forensic identification procedures. PCR technology can be used to amplify DNA sequences taken from very small biological samples, such as a single hair follicle, body fluids (eg. blood, saliva, or semen). The amplified sequence can then be compared to a standard allowing identification of the origin of the sample.

The receptor polynucleotides can thus be used to provide polynucleotide reagents, e.g., PCR primers, targeted to specific loci in the human genome, which can enhance the reliability of DNA-based forensic identifications by, for example, providing another "identification marker" (i.e. another DNA sequence that is unique to a particular individual). As described above, actual base sequence information can be used for identification as an accurate alternative to patterns formed by restriction enzyme generated fragments. Sequences targeted to the noncoding region are particularly useful since greater polymorphism occurs in the noncoding regions, making it easier to differentiate individuals using this technique.

The receptor polynucleotides can further be used to provide polynucleotide reagents, e.g., labeled or labelable probes which can be used in, for example, an *in situ* hybridization technique, to identify a specific tissue. This is useful in cases in which a forensic pathologist is presented with a tissue of unknown origin. Panels of receptor probes can be used to identify tissue by species and/or by organ type.

In a similar fashion, these primers and probes can be used to screen tissue culture for contamination (i.e. screen for the presence of a mixture of different types of cells in a culture).

Alternatively, the receptor polynucleotides can be used directly to block transcription or translation of receptor gene sequences by means of antisense or ribozyme constructs. Thus, in a disorder characterized by abnormally high or undesirable receptor gene expression, nucleic acids can be directly used for treatment.

The receptor polynucleotides are thus useful as antisense constructs to control receptor gene expression in cells, tissues, and organisms. A DNA antisense polynucleotide is designed to be complementary to a region of the gene involved in transcription, preventing transcription and hence production of receptor protein. An antisense RNA or DNA polynucleotide would hybridize to the mRNA and thus block translation of mRNA into receptor protein.

Examples of antisense molecules useful to inhibit nucleic acid expression include antisense molecules complementary to a fragment of the 5' untranslated region of SEQ ID NO 2 which also includes the start codon and antisense molecules which are complementary to a fragment of the 3' untranslated region of SEQ ID NO 2.

Alternatively, a class of antisense molecules can be used to inactivate mRNA in order to decrease expression of receptor nucleic acid. Accordingly, these molecules can treat a disorder characterized by abnormal or undesired receptor nucleic acid expression. This technique involves cleavage by means of ribozymes containing nucleotide sequences complementary to one or more regions in the mRNA that attenuate the ability of the mRNA to be translated. Possible regions include coding regions and particularly coding regions corresponding to the catalytic and other functional activities of the receptor protein, such as ligand binding.

The receptor polynucleotides also provide vectors for gene therapy in patients containing cells that are aberrant in receptor gene expression. Thus, recombinant cells, which include the patient's cells that have been engineered *ex vivo* and returned to the patient, are introduced into an individual where the cells produce the desired receptor protein to treat the individual.

The invention also encompasses kits for detecting the presence of a receptor nucleic acid in a biological sample. For example, the kit can comprise reagents such as a labeled or labelable nucleic acid or agent capable of detecting receptor nucleic acid in a biological sample; means for determining the amount of receptor nucleic acid in the sample; and means for comparing the amount of receptor nucleic acid in the sample with a standard. The compound or agent can be packaged in a suitable container. The kit can further comprise instructions for using the kit to detect receptor mRNA or DNA.

The above disclosure also applies to the sequence shown in SEQ ID NO 4.

### Vectors/host cells

The invention also provides vectors containing the receptor polynucleotides. The term "vector" refers to a vehicle, preferably a nucleic acid molecule, that can transport the receptor polynucleotides. When the vector is a nucleic acid molecule, the receptor polynucleotides are covalently linked to the vector nucleic acid. With this aspect of the invention, the vector includes a plasmid, single or double stranded phage, a single or double stranded RNA or DNA viral vector, or artificial chromosome, such as a BAC, PAC, YAC, OR MAC.

A vector can be maintained in the host cell as an extrachromosomal element where it replicates and produces additional copies of the receptor polynucleotides. Alternatively, the vector may integrate into the host cell genome and produce additional copies of the receptor polynucleotides when the host cell replicates.

The invention provides vectors for the maintenance (cloning vectors) or vectors for expression (expression vectors) of the receptor polynucleotides. The vectors can function in procaryotic or eukaryotic cells or in both (shuttle vectors).

Expression vectors contain cis-acting regulatory regions that are operably linked in the vector to the receptor polynucleotides such that transcription of the polynucleotides is allowed in a host cell. The polynucleotides can be introduced into the host cell with a separate polynucleotide capable of affecting transcription. Thus, the second polynucleotide may provide a trans-acting factor interacting with the cis-regulatory control region to allow transcription of the receptor polynucleotides from the vector. Alternatively, a trans-acting factor may be supplied by the host cell. Finally, a trans-acting factor can be produced from the vector itself.

It is understood, however, that in some embodiments, transcription and/or translation of the receptor polynucleotides can occur in a cell-free system.

The regulatory sequence to which the polynucleotides described herein can be operably linked include promoters for directing mRNA transcription. These include, but are not limited to, the left promoter from bacteriophage λ, the lac, TRP, and TAC promoters from *E. coli,* the early and late promoters from SV40, the CMV immediate early promoter, the adenovirus early and late promoters, and retrovirus long-terminal repeats.

In addition to control regions that promote transcription, expression vectors may also include regions that modulate transcription, such as repressor binding sites and enhancers. Examples include the SV40 enhancer, the cytomegalovirus immediate early enhancer, polyoma enhancer, adenovirus enhancers, and retrovirus LTR enhancers.

In addition to containing sites for transcription initiation and control, expression vectors can also contain sequences necessary for transcription termination and, in the transcribed region a ribosome binding site for translation. Other regulatory control elements for expression include initiation and termination codons as well as polyadenylation signals. The person of ordinary skill in the art would be aware of the numerous regulatory sequences that are useful in expression vectors. Such regulatory sequences are described, for example, in Sambrook *et al., Molecular Cloning. A Laboratory Manual. 2nd ed.,* Cold Spring Harbor Laboratory Press, Cold Spring Harbor, NY, (1989).

A variety of expression vectors can be used to express a receptor polynucleotide. Such vectors include chromosomal, episomal, and virus-derived vectors, for example vectors derived from bacterial plasmids, from bacteriophage, from yeast episomes, from yeast chromosomal elements, including yeast artificial chromosomes, from viruses such as baculoviruses, papovaviruses such as SV40, Vaccinia viruses, adenoviruses, poxviruses, pseudorabies viruses, and retroviruses. Vectors may also be derived from combinations of these sources such as those derived from plasmid and bacteriophage genetic elements, eg. cosmids and phagemids. Appropriate cloning and expression vectors for prokaryotic and eukaryotic hosts are described in Sambrook *et al., Molecular Cloning: A Laboratory Manual. 2nd ed.,* Cold Spring Harbor Laboratory Press, Cold Spring Harbor, NY, (1989).

The regulatory sequence may provide constitutive expression in one or more host cells (i.e. tissue specific) or may provide for inducible expression in one or more cell types such as by temperature, nutrient additive, or exogenous factor such as a hormone or other ligand. A variety of vectors providing for constitutive and inducible expression in prokaryotic and eukaryotic hosts are well known to those of ordinary skill in the art.

The receptor polynucleotides can be inserted into the vector nucleic acid by well-known methodology. Generally, the DNA sequence that will ultimately be expressed is joined to an expression vector by cleaving the DNA sequence and the expression vector with one or more restriction enzymes and then ligating the fragments together. Procedures for restriction enzyme digestion and ligation are well known to those of ordinary skill in the art.

The vector containing the appropriate polynucleotide can be introduced into an appropriate host cell for propagation or expression using well-known techniques. Bacterial cells include, but are not limited to, *E coli, Streptomyces,* and *Salmonella typhimurium.* Eukaryotic cells include, but are not limited to, yeast, insect cells such as *Drosophila,* animal cells such as COS and CHO cells, and plant cells.

As described herein, it may be desirable to express the polypeptide as a fusion protein. Accordingly, the invention provides fusion vectors that allow for the production of the receptor polypeptides. Fusion vectors can increase the expression of a recombinant protein, increase the solubility of the recombinant protein, and aid in the purification of the protein by acting for example as a ligand for affinity purification. A proteolytic cleavage site may be introduced at the junction of the fusion moiety so that the desired polypeptide can ultimately be separated from the fusion moiety. Proteolytic enzymes include, but are not limited to, factor Xa, thrombin, and enterokinase. Typical fusion expression vectors include pGEX (Smith *et al., Gene 67*:31-40 (1988)), pMAL (New England Biolabs, Beverly, MA) and pRIT5 (Pharmacia, Piscataway, NJ) which fuse glutathione S-transferase (GST), maltose E binding protein, or protein A, respectively, to the target recombinant protein. Examples of suitable inducible non-fusion *E. coli* expression vectors include pTrc (Amann *et al., Gene 69*:301-315 (1988)) and pET 11d (Studier *et al., Gene Expression Technology: Methods in Enzymology 185*:60-89 (1990)).

Recombinant protein expression can be maximized in a host bacteria by providing a genetic background wherein the host cell has an impaired capacity to proteolytically cleave the recombinant protein. (Gottesman, S., *Gene Expression Technology: Methods in Enzymology* 185, Academic Press, San Diego, California (1990) 119-128). Alternatively, the sequence of the polynucleotide of interest can be altered to provide preferential codon usage for a specific host cell, for example *E. coli.* (Wada *et al., Nucleic Acids Res. 20*:2111-2118 (1992)).

The receptor polynucleotides can also be expressed by expression vectors that are operative in yeast. Examples of vectors for expression in yeast e.g., *S. cerevisiae* include pYepSecl (Baldari, *et al., EMBO J. 6*:229-234 (1987)), pMFa (Kurjan *et al., Cell 30*:933-943(1982)), pJRY88 (Schultz *et al., Gene 54*:113-123 (1987)), and pYES2 (Invitrogen Corporation, San Diego, CA).

The receptor polynucleotides can also be expressed in insect cells using, for example, baculovirus expression vectors. Baculovirus vectors available for expression of proteins in cultured insect cells (e.g., Sf 9 cells) include the pAc series (Smith *et al., Mol. Cell Biol. 3*:2156-2165 (1983)) and the pVL series (Lucklow *et al., Virology 170*:31-39 (1989)).

In certain embodiments of the invention, the polynucleotides described herein are expressed in mammalian cells using mammalian expression vectors. Examples of mammalian expression vectors include pCDM8 (Seed, B. *Nature 329*:840(1987)) and pMT2PC (Kaufman *et al., EMBO J. 6*:187-195 (1987)).

The expression vectors listed herein are provided by way of example only of the well-known vectors available to those of ordinary skill in the art that would be useful to express the receptor polynucleotides. The person of ordinary skill in the art would be aware of other vectors suitable for maintenance propagation or expression of the polynucleotides described herein. These are found for example in Sambrook, J., Fritsh, E. F., and Maniatis, T. *Molecular Cloning: A Laboratory Manual. 2nd, ed, Cold Spring Harbor Laboratory,* Cold Spring Harbor Laboratory Press, Cold Spring Harbor, NY, 1989.

The invention also encompasses vectors in which the nucleic acid sequences described herein are cloned into the vector in reverse orientation, but operably linked to a regulatory sequence that permits transcription of antisense RNA. Thus, an antisense transcript can be produced to all, or to a portion, of the polynucleotide sequences described herein, including both coding and non-coding regions. Expression of this antisense RNA is subject to each of the parameters described above in relation to expression of the sense RNA (regulatory sequences, constitutive or inducible expression, tissue-specific expression).

The invention also relates to recombinant host cells containing the vectors described herein. Host cells therefore include prokaryotic cells, lower eukaryotic cells such as yeast, other eukaryotic cells such as insect cells, and higher eukaryotic cells such as mammalian cells.

The recombinant host cells are prepared by introducing the vector constructs described herein into the cells by techniques readily available to the person of ordinary skill in the art. These include, but are not limited to, calcium phosphate transfection, DEAE-dextran-mediated transfection, cationic lipid-mediated transfection, electroporation, transduction, infection, lipofection, and other techniques such as those found in Sambrook, *et al.* (*Molecular Cloning: A Laboratory Manual. 2nd, ed, Cold Spring Harbor Laboratory,* Cold Spring Harbor Laboratory Press, Cold Spring Harbor, NY, 1989).

Host cells can contain more than one vector. Thus, different nucleotide sequences can be introduced on different vectors of the same cell. Similarly, the receptor polynucleotides can be introduced either alone or with other polynucleotides that are not related to the receptor polynucleotides such as those providing trans-acting factors for expression vectors. When more than one vector is introduced into a cell, the vectors can be introduced independently, co-introduced or joined to the receptor polynucleotide vector.

In the case of bacteriophage and viral vectors, these can be introduced into cells as packaged or encapsulated virus by standard procedures for infection and transduction. Viral vectors can be replication-competent or replication-defective. In the case in which viral replication is defective, replication will occur in host cells providing functions that complement the defects.

Vectors generally include selectable markers that enable the selection of the subpopulation of cells that contain the recombinant vector constructs. The marker can be contained in the same vector that contains the polynucleotides described herein or may be on a separate vector. Markers include tetracycline or ampicillin-resistance genes for prokaryotic host cells and dihydrofolate reductase or neomycin resistance for eukaryotic host cells. However, any marker that provides selection for a phenotypic trait will be effective.

While the mature proteins can be produced in bacteria, yeast, mammalian cells, and other cells under the control of the appropriate regulatory sequences, cell-free transcription and translation systems can also be used to produce these proteins using RNA derived from the DNA constructs described herein.

Where secretion of the polypeptide is desired, appropriate secretion signals are incorporated into the vector. The signal sequence can be endogenous to the receptor polypeptides or heterologous to these polypeptides.

Where the polypeptide is not secreted into the medium, the protein can be isolated from the host cell by standard disruption procedures, including freeze thaw, sonication, mechanical disruption, use of lysing agents and the like. The polypeptide can then be recovered and purified by well-known purification methods including ammonium sulfate precipitation, acid extraction, anion or cationic exchange chromatography, phosphocellulose chromatography, hydrophobic-interaction chromatography, affinity chromatography, hydroxylapatite chromatography, lectin chromatography, or high performance liquid chromatography.

It is also understood that depending upon the host cell in recombinant production of the polypeptides described herein, the polypeptides can have various glycosylation patterns, depending upon the cell, or maybe non-glycosylated as when produced in bacteria. In addition, the polypeptides may include an initial modified methionine in some cases as a result of a host-mediated process.

The above disclosure also applies to the sequences shown in SEQ ID NOS 3 and 4.

### Uses of vectors and host cells

The host cells expressing the polypeptides described herein, and particularly recombinant host cells, have a variety of uses. First, the cells are useful for producing receptor proteins or polypeptides that can be further purified to produce desired amounts of receptor protein or fragments. Thus, host cells containing expression vectors are useful for polypeptide production.

Host cells are also useful for conducting cell-based assays involving the receptor or receptor fragments. Thus, a recombinant host cell expressing a native receptor is useful to assay for compounds that stimulate or inhibit receptor function. This includes ligand binding, gene expression at the level of transcription or translation, G-protein interaction, and components of the signal transduction pathway.

Host cells are also useful for identifying receptor mutants in which these functions are affected. If the mutants naturally occur and give rise to a pathology, host cells containing the mutations are useful to assay compounds that have a desired effect on the mutant receptor (for example, stimulating or inhibiting function) which may not be indicated by their effect on the native receptor.

Recombinant host cells are also useful for expressing the chimeric polypeptides described herein to assess compounds that activate or suppress activation by means of a heterologous amino terminal extracellular domain (or other binding region). Alternatively, a heterologous region spanning the entire transmembrane domain (or parts thereof) can be used to assess the effect of a desired amino terminal extracellular domain (or other binding region) on any given host cell. In this embodiment, a region spanning the entire transmembrane domain (or parts thereof) compatible with the specific host cell is used to make the chimeric vector. Alternatively, a heterologous carboxy terminal intracellular, e.g., signal transduction, domain can be introduced into the host cell.

Further, mutant receptors can be designed in which one or more of the various functions is engineered to be increased or decreased (e.g., ligand binding or G-protein binding) and used to augment or replace receptor proteins in an individual. Thus, host cells can provide a therapeutic benefit by replacing an aberrant receptor or providing an aberrant receptor that provides a therapeutic result. In one embodiment, the cells provide receptors that are abnormally active.

In another embodiment, the cells provide receptors that are abnormally inactive. These receptors can compete with endogenous receptors in the individual.

In another embodiment, cells expressing receptors that cannot be activated, are introduced into an individual in order to compete with endogenous receptors for ligand. For example, in the case in which excessive ligand is part of a treatment modality, it may be necessary to inactivate this ligand at a specific point in treatment. Providing cells that compete for the ligand, but which cannot be affected by receptor activation would be beneficial.

Homologously recombinant host cells can also be produced that allow the *in situ* alteration of endogenous receptor polynucleotide sequences in a host cell genome. This technology is more fully described in WO 93/09222, WO 91/12650 and U.S. 5,641,670. Briefly, specific polynucleotide sequences corresponding to the receptor polynucleotides or sequences proximal or distal to a receptor gene are allowed to integrate into a host cell genome by homologous recombination where expression of the gene can be affected. In one embodiment, regulatory sequences are introduced that either increase or decrease expression of an endogenous sequence. Accordingly, a receptor protein can be produced in a cell not normally producing it, or increased expression of receptor protein can result in a cell normally producing the protein at a specific level. Alternatively, the entire gene can be deleted. Still further, specific mutations can be introduced into any desired region of the gene to produce mutant receptor proteins. Such mutations could be introduced, for example, into the specific functional regions such as the ligand-binding site or the G-protein binding site.

In one embodiment, the host cell can be a fertilized oocyte or embryonic stem cell that can be used to produce a transgenic animal containing the altered receptor gene. Alternatively, the host cell can be a stem cell or other early tissue precursor that gives rise to a specific subset of cells and can be used to produce transgenic tissues in an animal. See also Thomas *et al., Cell 51*:503 (1987) for a description of homologous recombination vectors. The vector is introduced into an embryonic stem cell line (e.g., by electroporation) and cells in which the introduced gene has homologously recombined with the endogenous receptor gene is selected (see e.g., Li, E. *et al., Cell 69*:915 (1992)). The selected cells are then injected into a blastocyst of an animal (e.g., a mouse) to form aggregation chimeras (see e.g., Bradley, A. in *Teratocarcinomas and Embryonic Stem Cells: A Practical Approach,* E.J. Robertson, ed. (IRL, Oxford, 1987) pp. 113-152). A chimeric embryo can then be implanted into a suitable pseudopregnant female foster animal and the embryo brought to term. Progeny harboring the homologously recombined DNA in their germ cells can be used to breed animals in which all cells of the animal contain the homologously recombined DNA by germline transmission of the transgene. Methods for constructing homologous recombination vectors and homologous recombinant animals are described further in Bradley, A. (1991) *Current Opinion in Biotechnology* 2:823-829 and in PCT International Publication Nos. WO 90/11354; WO 91/01140; and WO 93/04169.

The genetically engineered host cells can be used to produce non-human transgenic animals. A transgenic animal is preferably a mammal, for example a rodent, such as a rat or mouse, in which one or more of the cells of the animal include a transgene. A transgene is exogenous DNA which is integrated into the genome of a cell from which a transgenic animal develops and which remains in the genome of the mature animal in one or more cell types or tissues of the transgenic animal. These animals are useful for studying the function of a receptor protein and identifying and evaluating modulators of receptor protein activity.

Other examples of transgenic animals include non-human primates, sheep, dogs, cows, goats, chickens, and amphibians.

In one embodiment, a host cell is a fertilized oocyte or an embryonic stem cell into which receptor polynucleotide sequences have been introduced.

A transgenic animal can be produced by introducing nucleic acid into the male pronuclei of a fertilized oocyte, e.g., by microinjection, retroviral infection, and allowing the oocyte to develop in a pseudopregnant female foster animal. Any of the receptor nucleotide sequences can be introduced as a transgene into the genome of a non-human animal, such as a mouse.

Any of the regulatory or other sequences useful in expression vectors can form part of the transgenic sequence. This includes intronic sequences and polyadenylation signals, if not already included. A tissue-specific regulatory sequence(s) can be operably linked to the transgene to direct expression of the receptor protein to particular cells.

Methods for generating transgenic animals via embryo manipulation and microinjection, particularly animals such as mice, have become conventional in the art and are described, for example, in U.S. Patent Nos. 4,736,866 and 4,870,009, both by Leder *et al.,* U.S. Patent No. 4,873,191 by Wagner *et al.* and in Hogan, B., *Manipulating the Mouse Embryo,* (Cold Spring Harbor Laboratory Press, Cold Spring Harbor, N.Y., 1986). Similar methods are used for production of other transgenic animals. A transgenic founder animal can be identified based upon the presence of the transgene in its genome and/or expression of transgenic mRNA in tissues or cells of the animals. A transgenic founder animal can then be used to breed additional animals carrying the transgene. Moreover, transgenic animals carrying a transgene can further be bred to other transgenic animals carrying other transgenes. A transgenic animal also includes animals in which the entire animal or tissues in the animal have been produced using the homologously recombinant host cells described herein.

In another embodiment, transgenic non-human animals can be produced which contain selected systems which allow for regulated expression of the transgene. One example of such a system is the *cre*/*loxP* recombinase system of bacteriophage P1. For a description of the *cre*/*loxP* recombinase system, see, e.g., Lakso *et al. PNAS 89*:6232-6236 (1992). Another example of a recombinase system is the FLP recombinase system of *S. cerevisiae* (O'Gorman *et al. Science 251*:1351-1355 (1991). If a *cre*/*loxP* recombinase system is used to regulate expression of the transgene, animals containing transgenes encoding both the *Cre* recombinase and a selected protein is required. Such animals can be provided through the construction of "double" transgenic animals, e.g., by mating two transgenic animals, one containing a transgene encoding a selected protein and the other containing a transgene encoding a recombinase.

Clones of the non-human transgenic animals described herein can also be produced according to the methods described in Wilmut, I. *et al. Nature 385*:810-813 (1997) and PCT International Publication Nos. WO 97/07668 and WO 97/07669. In brief, a cell, e.g., a somatic cell, from the transgenic animal can be isolated and induced to exit the growth cycle and enter Go phase. The quiescent cell can then be fused, e.g., through the use of electrical pulses, to an enucleated oocyte from an animal of the same species from which the quiescent cell is isolated. The reconstructed oocyte is then cultured such that it develops to morula or blastocyst and then transferred to pseudopregnant female foster animal. The offspring born of this female foster animal will be a clone of the animal from which the cell, e.g., the somatic cell, is isolated.

Transgenic animals containing recombinant cells that express the polypeptides described herein are useful to conduct the assays described herein in an *in vivo* context. Accordingly, the various physiological factors that are present *in vivo* and that could effect ligand binding, receptor activation, and signal transduction, may not be evident from *in vitro* cell-free or cell-based assays. Accordingly, it is useful to provide non-human transgenic animals to assay *in vivo* receptor function, including ligand interaction, the effect of specific mutant receptors on receptor function and ligand interaction, and the effect of chimeric receptors. It is also possible to assess the effect of null mutations, that is mutations that substantially or completely eliminate one or more receptor functions.

The above disclosure also applies to the sequences shown in SEQ ID NOS 3 and 4.

### Pharmaceutical compositions

The receptor nucleic acid molecules, protein (particularly fragments such as the amino terminal extracellular domain), modulators of the protein, and antibodies (also referred to herein as "active compounds") can be incorporated into pharmaceutical compositions suitable for administration to a subject, e.g., a human. Such compositions typically comprise the nucleic acid molecule, protein, modulator, or antibody and a pharmaceutically acceptable carrier

As used herein the language "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, such media can be used in the compositions of the invention. Supplementary active compounds can also be incorporated into the compositions. A pharmaceutical composition of the invention is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. PH can be adjusted with acids or bases, such as hydrochloric acid or sodium hydroxide. The parenteral preparation can be enclosed in ampules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor ELJ (BASF, Parsippany, NJ) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (e.g., a receptor protein or anti-receptor antibody) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-fihered solution thereof.

Oral compositions generally include an inert diluent or an edible carrier. They can be enclosed in gelatin capsules or compressed into tablets. For oral administration, the agent can be contained in enteric forms to survive the stomach or further coated or mixed to be released in a particular region of the GI tract by known methods. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

The nucleic acid molecules of the invention can be inserted into vectors and used as gene therapy vectors. Gene therapy vectors can be delivered to a subject by, for example, intravenous injection, local administration (U.S. 5,328,470) or by stereotactic injection (see e.g., Chen *et al., PNAS 91*:3054-3057 (1994)). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g. retroviral vectors, the pharmaceutical preparation can include one or more cells which produce the gene delivery system.

The pharmaceutical compositions can be included in a container, pack, or dispenser together with instructions for administration.

The disclosure above also applies to the sequences shown in SEQ ID NOS 3 and 4.

## Claims

1. A method for identifying an agent that modulates the level or activity of a polypeptide in a cell or that interacts with a polypeptide in a cell, wherein said polypeptide is selected from:
(a) the amino acid sequence shown in SEQ ID NO: 1 or 3;
(b) the amino acid sequence of an alleic variant of the amino acid sequence shown in SEQ ID NO: 1 or 3;
(c) the amino acid sequence of a sequence variant of the amino acid sequence shown in SEQ ID NO: 1 or 3, wherein the sequence variant is encoded by a nucleic acid molecule hybridizing to the nucleic acid molecule shown in SEQ ID NO: 2 or 4, respectively, under stringent conditions;
(d) a fragment of the amino acid sequence shown in SEQ ID NO: 1 or 3, wherein the fragment comprises at least 10 contiguous amino acids;
(e) the amino acid sequence of the mature receptor polypeptide from about amino acid 6 to about amino acid 342, shown in SEQ ID NO: I or 3;
(f) the amino acid sequence of the polypeptide shown in SEQ ID NO: 1 or 3, from about amino acid 1 to about amino acid 342; and
(g) the amino acid sequence of an epitope bearing region of any one of the polypeptides of (a)-(f); and said method comprises:
(i) contacting said agent with a cell capable of expressing said polypeptide such that said polypeptide level or activity can be modulated in said cell by said agent, wherein said cell is derived from brain, CD34⁺ cells, B cells, skeletal muscle, lymph node, spleen, thymus, liver, tonsil, colon, heart, granulocytes, arythroblasts or pancreas; and
(ii) measuring said polypeptide level or activity.

2. The method of claim 1 wherein said agent modulates an interaction between said polypeptide and a target molecule for said polypeptide, step (i) is carried out under conditions that allow said polypeptide to interact with said target molecule, and step (ii) comprises detecting the formation of a complex between said polypeptide and said target molecule or detecting activity of said polypeptide as a result of interaction of said polypeptide with said target molecule.

3. The method of claim 1 or 2, wherein said agent increases the level or activity of said polypeptide.

4. The method of claim 1 or 2, wherein said agent decreases the level or activity of said polypeptide.

5. The method of any one of the preceding claims, wherein step (ii) comprises:
(1) adding competing polypeptide that can interact with said agent; and
(2) comparing the amount of interaction between said agent and said polypeptide to the amount of interaction in the absence of said competing polypeptide.

6. The method of any one of the preceding claims, wherein said brain cell is a glial cell.

7. The method of any one of the preceding claims wherein said cell is *in vivo* and wherein said cell is not in a human.

8. The method of claim 7 wherein said cell is in a non-human transgenic or non-transgenic animal.

9. The method of any one of claims 1 to 6 wherein said cell is *in vitro*.

10. The method of claim 9 wherein said cell has been disrupted.

11. The method of claim 9 wherein said cell is in a biopsy or is a naturally-occurring or recombinant cell in cell culture.

12. The method of any one of the preceding claims wherein said agent is selected from a peptide, a phosphopeptide, an antibody, an organic molecule and an inorganic molecule.

13. A method for detecting the presence of a polypeptide in a sample, said method comprising;
(i) contacting said sample with an antibody that specifically allows detection of the presence of the polypeptide in the sample; and
(ii) detecting the presence of the polypeptide, wherein said polypeptide is selected from:
(a) the amino acid sequence shown in SEQ ID NO: 1 or 3;
(b) the amino acid sequence of an alleic variant of the amino acid sequence shown in SEQ ID NO: 1 or 3;
(c) the amino acid sequence of a sequence variant of the amino acid sequence shown in SEQ ID NO: 1 or 3, wherein the sequence variant is encoded by a nucleic acid molecule hybridizing to the nucleic acid molecule shown in SEQ ID NO: 2 or 4, respectively, under stringent conditions;
(d) a fragment of the amino acid sequence shown in SEQ ID NO: 1 or 3, wherein the fragment comprises at least 10 contiguous amino acids;
(e) the amino acid sequence of the mature receptor polypeptide from about amino acid 6 to about amino acid 342, shown in SEQ ID NO: 1 or 3;
(f) the amino acid sequence of the polypeptide shown in SEQ ID NO: 1 or 3, from about amino acid 1 to about amino acid 342;
(g) the amino acid sequence of an epitope bearing region of any one of the polypeptides of (a)-(f);
and wherein said sample is derived from a cell selected from brain, CD34⁺, B, skeletal muscle, lymph node, spleen, thymus, liver, tonsil, colon, heart, granulocyte, erthroblast and pancreatic cells.

14. The method of claim 13 wherein said detecting is in a cell derived from a subject having a disorder involving said cell.

15. A method for identifying an agent that modulates the level or activity of a nucleic acid molecule in a cell or that interacts with a nucleic acid molecule in a cell, wherein said nucleic acid molecule has a nucleic acid sequence selected from:
(a) the nucleotide sequence shown in SEQ ID NO: 2 or 4;
(b) a nucleotide sequence encoding the amino sequence shown in SEQ ID NO: 1 or 3;
(c) a nucleotide sequence complementary to any of the nucleotide sequences in (a) or (b);
(d) a nucleotide sequence encoding an amino acid sequence of a sequence variant of the amino acid sequence shown in SEQ ID NO: 1 or 3 that hybridizes to the nucleotide sequence shown in SEQ ID NO: 2 or 4, respectively, under stringent conditions;
(e) a nucleotide sequence complementary to the nucleotide sequence in (d);
(f) a nucleotide sequence encoding a fragment of the amino acid sequence shown in SEQ ID NO: 1 or 3, wherein the fragment comprises at least 10 contiguous amino acids; and
(g) a nucleotide sequence complementary to the nucleotide sequence in (f);
and said method comprises:
(i) contacting said agent with a cell capable of expressing said nucleic acid molecule such that said nucleic acid molecule level or activity can be modulated in said cell by said agent, wherein said cell is derived from brain, CD34⁺ cells, B cells, skeletal muscle, lymph node, spleen, thymus, liver, tonsil, colon, heart, granulocytes, erythroblasts or pancreas and;
(ii) measuring said nucleic acid molecule level or activity.

16. A method for detecting the presence of a nucleic acid molecule in a sample, said method comprising:
(i) contacting said sample with an agent that specifically allows detection of the presence of the nucleic acid molecule in the sample and;
(ii) detecting the presence of the nucleic acid molecule or fragment thereof, wherein the nucleic acid molecule has a nucleotide sequence selected from:
(a) the nucleotide sequence shown in SEQ ID NO: 2 or 4;
(b) a nucleotide sequence encoding the amino acid sequence shown in SEQ ID NO: 1 or 3;
(c) a nucleotide sequence complementary to any of the nucleotide sequences in (a) or (b);
(d) a nucleotide sequence encoding an amino acid sequence of a sequence variant of the amino acid sequence shown in SEQ ID NO: 1 or 3 that hybridizes to the nucleotide sequence shown in SEQ ID NO: 2 or 4, respectively, under stringent conditions;
(e) a nucleotide sequence complementary to the nucleotide sequence in (d);
(f) a nucleotide sequence encoding a fragment of the amino acid sequence shown in SEQ ID NO: 1 or 3, wherein the fragment comprises at least 10 contiguous amino acids; and
(g) a nucleotide sequence complementary to the nucleotide sequence in (f);
wherein said sample is derived from a tissue selected from brain, CD34⁺ cells, B cells, skeletal muscle, lymph node, spleen, thymus, liver, tonsil, colon, heart, granulocytes, erythroblasts and pancreas, and wherein said agent comprises an oligonucleotide that hybridizes to any one of the nucleic acid sequences of (a)-(g) under stringent conditions and step (ii) comprises determining whether the oligonucleotide binds to the nucleic acid sequence in the sample.

17. The method of claim 16 wherein the nucleic acid is mRNA.

## Patentansprüche

1. Verfahren zur Identifizierung eines Mittels, das das Level oder die Aktivität eines Polypeptids in einer Zelle moduliert, oder das mit einem Polypeptid in einer Zelle in Wechselwirkung tritt, wobei das Polypeptid ausgewählt ist aus:
(a) der in SEQ ID NO: 1 oder 3 dargestellten Aminosäure-Sequenz;
(b) der Aminosäure-Sequenz einer Allel-Variante der in SEQ ID NO: 1 oder 3 dargestellten Aminosäure-Sequenz;
(c) der Aminosäure-Sequenz oder einer Sequenzvariante der in SEQ ID NO: 1 oder 3 dargestellten Aminosäure-Sequenz, wobei die Sequenzvariante durch ein Nucleinsäuremolekül codiert wird, das an das in SEQ ID NO: 2 bzw. 4 dargestellte Nuclsäuremolekül unter strengen Bedingungen hybridisiert;
(d) einem Fragment der in SEQ ID NO: 1 oder 3 dargestellten Aminosäure-Sequenz, worin das Fragment mindestens 10 angrenzende Aminosäuren aufweist;
(e) der Aminosäure-Sequenz des reifen Rezeptor-Polypeptids von ca. Aminosäure 6 bis ca. Aminosäure 342, dargestellt in SEQ ID NO: 1 oder 3;
(f) der Aminosäure-Sequenz des in SEQ ID NO: 1 oder 3 dargestellten Polypeptids von ca. mindestens Aminosäure 1 bis ca. Aminosäure 342; und
(g) der Aminosäure-Sequenz eines Epitops, das eine Region eines der Polypeptide von (a) bis (f) trägt;
und das Verfahren umfasst:
(i) in Kontakt bringen des Mittels mit einer Zelle, die dazu fähig ist, das Polypeptid so zu exprimieren, dass das Level oder die Aktivität des Polypeptids in dieser Zelle durch das Mittel moduliert werden kann, wobei die Zelle abgeleitet ist aus Gehirn, CD34⁺-Zellen, B-Zellen, Skelettmuskel, Lymphknoten, Milz, Thymus, Leber, Mandeln, Kolon, Herz, Ganulozyten, Erythroblasten oder Pankreas; und
(ii) Messen des Levels oder der Aktivität des Polypeptids.

2. Verfahren nach Anspruch 1, worin das Mittel eine Wechselwirkung zwischen dem Polypeptid und einem Zielmolekül für das Polypeptid moduliert, Stufe (i) unter Bedingungen ausgeführt wird, die es dem Polypeptid ermöglichen, mit dem Zielmolekül in Wechselwirkung zu treten, und Stufe (ii) die Bestimmung der Bildung eines Komplexes zwischen dem Polypeptid und dem Zielmolekül oder die Bestimmung der Aktivität des Polypeptids als Ergebnis der Wechselwirkung des Polypeptids mit dem Zielmolekül umfasst.

3. Verfahren nach Anspruch 1 oder 2, worin das Mittel das Level oder die Aktivität des Polypeptids erhöht.

4. Verfahren nach Anspruch 1o der 2, worin das Mittel das Level oder die Aktivität des Polypeptids verringert.

5. Verfahren nach einem der vorhergehenden Ansprüche, worin Stufe (ii) aufweist:
(1) Zugeben eines konkurrierenden Polypeptids, das mit dem Mittel in Wechselwirkung treten kann; und
(2) Vergleichen des Grades der Wechselwirkung zwischen mit dem Mittel und dem Polypeptid mit dem Grad der Wechselwirkung in Abwesenheit des konkurrierenden Polypeptids.

6. Verfahren nach einem der vorhergehenden Ansprüche, worin die Gehirnzelle eine Gliazelle ist.

7. Verfahren nach einem der vorhergehenden Ansprüche, worin die Zelle *in vivo* und nicht in einem Menschen vorhanden ist.

8. Verfahren nach Anspruch 7, worin die Zelle in einem nicht-menschlichen transgenen oder nicht-transgenen Tier vorhanden ist.

9. Verfahren nach einem der Ansprüche 1 bis 6, worin die Zelle *in vitro* vorhanden ist.

10. Verfahren nach Anspruch 9, worin die Zelle zerstört wurde.

11. Verfahren nach Anspruch 9, worin die Zelle aus einer Biopsie resultiert oder eine natürlich vorkommende oder rekombinante Zelle in einer Zellkultur ist.

12. Verfahren nach einem der vorhergehenden Ansprüche, worin das Mittel ausgewählt ist aus einem Peptid, einem Phosphopeptid, einem Antikörper, einem organischen Molekül und einem anorganischen Molekül.

13. Verfahren zur Bestimmung des Vorhandenseins eines Polypeptids in einer Probe, wobei das Verfahren aufweist:
(i) in Kontakt bringen der Probe mit einem Antikörper, der die spezifische Bestimmung der Gegenwart des Polypeptids in der Probe ermöglicht; und
(ii) Bestimmen der Gegenwart des Polypeptids, wobei das Polypeptid ausgewählt ist aus:
(a) der in SEQ ID NO: 1 oder 3 dargestellten Aminosäure-Sequenz;
(b) der Aminosäure-Sequenz einer Allel-Variante der in SEQ ID NO: 1 oder 3 dargestellten Aminosäure-Sequenz;
(c) der Aminosäure-Sequenz oder einer Sequenzvariante der in SEQ ID NO: 1 oder 3 dargestellten Aminosäure-Sequenz, wobei die Sequenzvariante durch ein Nucleinsäuremolekül codiert wird, das an das in SEQ ID NO: 2 bzw. 4 dargestellte Nuclsäuremolekül unter strengen Bedingungen hybridisiert;
(d) einem Fragment der in SEQ ID NO: 1 oder 3 dargestellten Aminosäure-Sequenz, worin das Fragment mindestens 10 angrenzende Aminosäuren aufweist;
(e) der Aminosäure-Sequenz des reifen Rezeptor-Polypeptids von ca. Aminosäure 6 bis ca. Aminosäure 342, dargestellt in SEQ ID NO: 1 oder 3;
(f) der Aminosäure-Sequenz des in SEQ ID NO: 1 oder 3 dargestellten Polypeptids von ca. mindestens Aminosäure 1 bis ca. Aminosäure 342; und
(g) der Aminosäure-Sequenz eines Epitops, das eine Region eines der Polypeptide von (a) bis (f) trägt;
und worin die Probe aus einer Zelle abgeleitet ist, die ausgewählt ist aus Gehirn, CD34⁺, B, Skelettmuskel, Lymphknoten, Milz, Thymus, Leber, Mandeln, Kolon, Herz, Granulozyten, Erithroblasten und Pankreaszellen.

14. Verfahren nach Anspruch 13, worin die Bestimmung in einer Zelle durchgeführt wird, die sich von einem Subjekt ableitet, das eine Erkrankung aufweist, die mit dieser Zelle zu tun hat.

15. Verfahren zur Identifizierung eines Mittels, das das Level oder die Aktivität eines Nucleinsäuremoleküls in einer Zelle moduliert, oder das mit einem Nucleinsäuremolekül in einer Zelle in Wechselwirkung tritt, wobei das Nucleinsäuremolekül eine Nulceinsäure-Sequenz aufweist, die ausgewählt ist aus:
(a) der in SEQ ID NO: 2 oder 4 dargestellten Nucleotid-Sequenz;
(b) einer Nucleotid-Sequenz, die die in SEQ ID NO: 1 oder 3 dargestellte Aminosäure-Sequenz codiert;
(c) einer zu einer der Nucleotid-Sequenzen in (a) oder (b) komplementären Nucleotid-Sequenz;
(d) einer Nucleotid-Sequenz, die eine Aminosäure-Sequenz einer Sequenzvariante der in SEQ ID NO: 1 oder 3 dargestellten Aminosäure-Sequenz codiert, die an die in SEQ ID NO: 2 bzw. 4 dargestellte Nucleotid-Sequenz unter strengen Bedingungen hybridisiert;
(e) einer Nucleotid-Sequenz, die zur Nucleotid-Sequenz in (d) komplementär ist;
(f) einer Nucleotid-Sequenz, die ein Fragment der in SEQ ID NO: 1 oder 3 dargestellten Aminosäure-Sequenz codiert, wobei das Fragment mindestens 10 angrenzende Aminosäuren aufweist; und
(g) einer Nucleotid-Sequenz, die zu der Nucleotid-Sequenz in (f) komplementär ist;
und das Verfahren umfasst:
(i) in Kontakt bringen des Mittels mit einer Zelle, die dazu fähig ist, das Nucleinsäuremolekül so zu exprimieren, dass das Level oder die Aktivität des Nucleinsäuremoleküls in der Zelle durch das Mittel moduliert werden kann, wobei die Zelle abgeleitet ist aus Gehirn, CD34⁺-Zellen, B-Zellen, Skelettmuskel, Lymphknoten, Milz, Thymus, Leber, Mandeln, Kolon, Herz, Granulozyten, Erythroblasten oder Pankreas, und
(ii) Messen des Levels oder der Aktivität des Nucleinsäuremoleküls.

16. Verfahren zur Bestimmung der Anwesenheit eines Nucleinsäuremoleküls in einer Probe, wobei das Verfahren umfasst:
(i) in Kontakt bringen der Probe mit einem Mittel, das eine spezifische Bestimmung der Anwesenheit des Nucleinsäuremoleküls in der Probe ermöglicht, und
(ii) Bestimmen der Anwesenheit des Nucleinsäuremoleküls oder Fragments davon, wobei das Nucleinsäuremolekül eine Nucleotid-Sequenz aufweist, die ausgewählt ist aus:
(a) der in SEQ ID NO: 2 oder 4 dargestellten Nucleotid-Sequenz,
(b) einer Nucleotid-Sequenz, die die in SEQ ID NO: 1 oder 3 dargestellte Aminosäure-Sequenz codiert;
(c) einer Nucleotid-Sequenz, die zu einer der Nucleotid-Sequenzen in (a) oder (b) komplementär ist;
(d) einer Nucleotid-Sequenz, die eine Aminosäure-Sequenz einer Sequenzvariante der in SEQ ID NO: 1 oder 3 dargestellten Aminosäure-Sequenz codiert, die an die in SEQ ID NO: 2 bzw. 4 dargestellte Nucleotid-Sequenz unter strengen Bedingungen hybridisiert;
(e) einer Nucleotid-Sequenz, die zur Nucleotid-Sequenz in (d) komplementär ist;
(f) einer Nucleotid-Sequenz, die ein Fragment der in SEQ ID NO: 1 oder 3 dargestellten Aminosäure-Sequenz codiert, wobei das Fragment mindestens 10 angrenzende Aminosäuren aufweist; und
(g) einer Nucleotid-Sequenz, die zur Nucleotid-Sequenz in (f) komplementär ist;
wobei die Probe abgeleitet ist aus einem Gewebe, ausgewählt aus Gehirn, CD34⁺-Zellen, B-Zellen, Skelettmuskel, Lymphknoten, Milz, Thymus, Leber, Mandeln, Kolon, Herz, Granulozyten, Erythroblasten und Pankreas, und wobei das Mittel ein Oligonucleotid aufweist, das an eine der Nucleotidsäure-Sequenzen von (a) bis (g) unter strengen Bedingungen hybridisiert, und Stufe (ii) das Bestimmen umfasst, ob das Oligonucleotid an die Nucleinsäure-Sequenz in der Probe bindet.

17. Verfahren nach Anspruch 16, worin die Nucleinsäure mRNA ist.

## Revendications

1. Procédé pour identifier un agent qui module le niveau ou l'activité d'un polypeptide dans une cellule ou qui interagit avec un polypeptide dans une cellule, dans lequel ledit polypeptide est sélectionné parmi :
(a) la séquence d'acides aminés représentée dans SEQ ID NO : 1 ou 3 ;
(b) la séquence d'acides aminés d'un variant allèlique de la séquence d'acides aminés représentée dans SEQ ID NO : 1 ou 3 ;
(c) la séquence d'acides aminés d'un variant de la séquence d'acides aminés représentée dans SEQ ID NO : 1 ou 3, dans laquelle le variant de séquence est codé par une molécule d'acide nucléique s'hybridant à la molécule d'acide nucléique représentée dans SEQ ID NO : 2 ou 4, respectivement, dans des conditions stringentes ;
(d) un fragment de la séquence d'acides aminés représentée dans SEQ ID NO : 1 ou 3, dans lequel le fragment comprend au moins 10 acides aminés contigus ;
(e) la séquence d'acides aminés du polypeptide récepteur mature, depuis environ l'acide aminé 6 jusqu'à environ l'acide aminé 342, représentée dans SEQ ID NO : 1 ou 3 ;
(f) la séquence d'acides aminés du polypeptide représentée dans SEQ ID NO : 1 ou 3, depuis environ l'acide aminé 1 jusqu'à environ l'acide aminé 342 ; et
(g) la séquence d'acides aminés d'une région porteuse d'un épitope de l'un quelconque des polypeptides de (a) à (f) ;
et ledit procédé comprend les étapes consistant à :
(i) mettre ledit agent en contact avec une cellule capable d'exprimer ledit polypeptide, de telle sorte que le niveau ou l'activité dudit polypeptide puisse être modulé dans ladite cellule par ledit agent, dans lequel ladite cellule provient du cerveau, de cellules CD34⁺, de lymphocytes B, de muscles squelettiques, de ganglions lymphatiques, de la rate, du thymus, du foie, des amygdales, du colon, du coeur, de granulocytes, d'érythroblastes ou du pancréas ; et
(ii) mesurer le niveau ou l'activité dudit polypeptide.

2. Procédé selon la revendication 1, dans lequel ledit agent module une interaction entre ledit polypeptide et une molécule cible pour ledit polypeptide, l'étape (i) est réalisée dans des conditions qui permettent audit polypeptide d'interagir avec ladite molécule cible, et l'étape (ii) comprend la détection de la formation d'un complexe entre ledit polypeptide et ladite molécule cible, ou la détection de l'activité dudit polypeptide en tant que résultat de l'interaction dudit polypeptide avec ladite molécule cible.

3. Procédé selon la revendication 1 ou 2, dans lequel ledit agent augmente le niveau ou l'activité dudit polypeptide.

4. Procédé selon la revendication 1 ou 2, dans lequel ledit agent diminue le niveau ou l'activité dudit polypeptide.

5. Procédé selon l'une quelconque des revendications précédentes, dans lequel l'étape (ii) comprend les étapes consistant à :
(1) ajouter un polypeptide concurrent pouvant interagir avec ledit agent ; et
(2) comparer le degré d'interaction entre ledit agent et ledit polypeptide au degré d'interaction en l'absence dudit polypeptide compétiteur.

6. Procédé selon l'une quelconque des revendications précédentes, dans lequel ladite cellule cérébrale est une cellule gliale.

7. Procédé selon l'une quelconque des revendications précédentes, dans lequel ladite cellule est *in vivo,* et dans lequel ladite cellule ne se trouve pas chez un humain.

8. Procédé selon la revendication 7, dans lequel ladite cellule se trouve chez un animal non humain, transgénique ou non transgénique.

9. Procédé selon l'une quelconque des revendications 1 à 6, dans lequel ladite cellule est *in vitro.*

10. Procédé selon la revendication 9, dans lequel ladite cellule a été fragmentée.

11. Procédé selon la revendication 9, dans lequel ladite cellule provient d'une biopsie, ou est une cellule d'origine naturelle ou recombinante dans une culture cellulaire.

12. Procédé selon l'une quelconque des revendications précédentes, dans lequel ledit agent est sélectionné parmi un peptide, un phosphopeptide, un anticorps, une molécule organique et une molécule inorganique.

13. Procédé pour détecter la présence d'un polypeptide dans un échantillon, ledit procédé comprenant les étapes consistant à :
(i) mettre ledit échantillon en contact avec un anticorps qui permet de détecter de façon spécifique la présence du polypeptide dans l'échantillon ; et
(ii) détecter la présence du polypeptide, ledit polypeptide étant sélectionné parmi :
(a) la séquence d'acides aminés représentée dans SEQ ID NO : 1 ou 3 ;
(b) la séquence d'acides aminés d'un variant allèlique de la séquence d'acides aminés représentée dans SEQ ID NO : 1 ou 3 ;
(c) la séquence d'acides aminés d'un variant de la séquence d'acides aminés représentée dans la séquence SEQ ID NO : 1 ou 3, dans laquelle le variant de séquence est codé par une molécule d'acide nucléique s'hybridant à la molécule d'acide nucléique représentée dans SEQ ID NO : 2 ou 4, respectivement, dans des conditions stringentes ;
(d) un fragment de la séquence d'acides aminés représentée dans SEQ ID NO : 1 ou 3, dans lequel le fragment comprend au moins 10 acides aminés contigus ;
(e) la séquence d'acides aminés du polypeptide récepteur mature, depuis environ l'acide aminé 6 jusqu'à environ l'acide aminé 342, représentée dans SEQ ID NO : 1 ou 3 ;
(f) la séquence d'acides aminés du polypeptide, représentée dans SEQ ID NO : 1 ou 3, depuis environ l'acide aminé 1 jusqu'à environ l'acide aminé 342 ;
(g) la séquence d'acides aminés d'une région porteuse d'un épitope de l'un quelconque des polypeptides de (a) à (f) ;
et dans lequel ledit échantillon provient d'une cellule sélectionnée parmi des cellules cérébrales, CD34⁺, des lymphocytes B, des cellules de muscles squelettiques, de ganglions lymphatiques, de rate, du thymus, du foie, des amygdales, du colon, du coeur, des granulocytes, des érythroblastes et des cellules pancréatiques.

14. Procédé selon la revendication 13, dans lequel ladite détection se fait dans une cellule provenant d'un sujet présentant un trouble impliquant ladite cellule.

15. Procédé pour identifier un agent qui module le niveau ou l'activité d'une molécule d'acide nucléique dans une cellule, ou qui interagit avec une molécule d'acide nucléique dans une cellule, ladite molécule d'acide nucléique ayant une séquence d'acides nucléiques sélectionnée parmi :
(a) la séquence de nucléotides représentée dans SEQ ID NO : 2 ou 4 ;
(b) une séquence de nucléotides codant pour la séquence d'acides aminés représentée dans SEQ ID NO : 1 ou 3 ;
(c) une séquence de nucléotides complémentaire à l'une quelconque des séquences de nucléotides en (a) ou (b) ;
(d) une séquence de nucléotides codant pour une séquence d'acides aminés d'un variant de la séquence d'acides aminés représentée dans SEQ ID NO : 1 ou 3, hybride de la séquence de nucléotides représentée dans la séquence SEQ ID NO : 2 ou 4, respectivement, dans des conditions stringentes ;
(e) une séquence de nucléotides complémentaire à la séquence de nucléotides en (d) ;
(f) une séquence de nucléotides codant pour un fragment de la séquence d'acides aminés représentée dans SEQ ID NO : 1 ou 3, dans laquelle le fragment comprend au moins 10 acides aminés contigus ; et
(g) une séquence de nucléotides complémentaire à la séquence des nucléotides en (f) ;
et ledit procédé comprend les étapes consistant à :
(i) mettre ledit agent en contact avec une cellule capable d'exprimer ladite molécule d'acide nucléique, de telle sorte que le niveau ou l'activité de ladite molécule d'acide nucléique puisse être régulé dans ladite cellule par ledit agent, ladite cellule provenant du cerveau, de cellules CD34⁺, de lymphocytes B, de muscles squelettiques, de ganglions lymphatiques, de la rate, du thymus, du foie, des amygdales, du colon, du coeur, de granulocytes, d'érythroblastes ou du pancréas, et ;
(ii) mesurer le niveau ou l'activité de ladite molécule d'acide nucléique.

16. Procédé pour détecter la présence d'une molécule d'acide nucléique dans un échantillon, ledit procédé comprenant les étapes consistant à :
(i) mettre ledit échantillon en contact avec un agent qui permet de détecter de façon spécifique la présence de la molécule d'acide nucléique dans l'échantillon et ;
(ii) détecter la présence de la molécule d'acide nucléique ou d'un fragment de celle-ci, la molécule d'acide nucléique ayant une séquence de nucléotides sélectionnée parmi :
(a) la séquence de nucléotides représentée dans SEQ ID NO : 2 ou 4 ;
(b) une séquence de nucléotides codant pour la séquence d'acides aminés représentée dans SEQ ID NO : 1 ou 3 ;
(c) une séquence de nucléotides complémentaire à l'une quelconque des séquences de nucléotides en (a) ou (b) ;
(d) une séquence de nucléotides codant pour une séquence d'acides aminés d'un variant de la séquence d'acides aminés représentée dans la séquence SEQ ID NO : 1 ou 3, qui s'hybride à la séquence de nucléotides représentée dans SEQ ID NO : 2 ou 4, respectivement, dans des conditions stringentes ;
(e) une séquence de nucléotides complémentaire à la séquence de nucléotides en (d) ;
(f) une séquence de nucléotides codant pour un fragment de la séquence d'acides aminés représentée dans SEQ ID NO : 1 ou 3, dans laquelle le fragment comprend au moins 10 acides aminés contigus ; et
(g) une séquence de nucléotides complémentaire à la séquence de nucléotides en (f)
dans laquelle ledit échantillon provient d'un tissu sélectionné parmi le cerveau, les cellules CD34⁺, les lymphocytes B, les muscles squelettiques, les ganglions lymphatiques, la rate, le thymus, le foie, les amygdales, le colon, le coeur, les granulocytes, les érythroblastes et le pancréas, et dans laquelle ledit agent comprend un oligonucléotide hybride de l'une quelconque des séquences d'acides nucléiques de (a) à (g) dans des conditions stringentes, et l'étape (ii) comprend l'étape consistant à déterminer si l'oligonucléotide se lie ou pas à la séquence d'acides nucléiques dans l'échantillon.

17. Procédé selon la revendication 16, dans lequel l'acide nucléique est l'ARNm.
